(19) Europäisches Patentamt
European Patent Office
Office européen des brevets

(11) EP 3 885 453 A1

(12) EUROPEAN PATENT APPLICATION

(43) Date of publication:
29.09.2021 Bulletin 2021/39

(51) Int Cl.:
*C12Q 1/6886* (2018.01)

(21) Application number: 20164829.2

(22) Date of filing: 23.03.2020

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
- HOFFMANN, Ralf Dieter
5656 AE Eindhoven (NL)
- VAN LIESHOUT, Ron Martinus Laurentius
5656 AE Eindhoven (NL)
- STOFFELS, Monique
5656 AE Eindhoven (NL)

(74) Representative: Philips Intellectual Property & Standards
High Tech Campus 5
5656 AE Eindhoven (NL)

# (54) PREDICTION OF RADIOTHERAPY RESPONSE FOR PROSTATE CANCER SUBJECT BASED ON DNA REPAIR GENES

(57) The invention relates to a method of predicting a response of a prostate cancer subject to radiotherapy, comprising determining or receiving the result of a determination of a gene expression profile for each of one or more DNA repair genes selected from the group consisting of: APTX, BRCA2, NUDT1, PMS2, and POLD2, said gene expression profile(s) being determined in a biological sample obtained from the subject, and determining the prediction of the radiotherapy response based on the gene expression profile(s) for the one or more DNA repair genes.

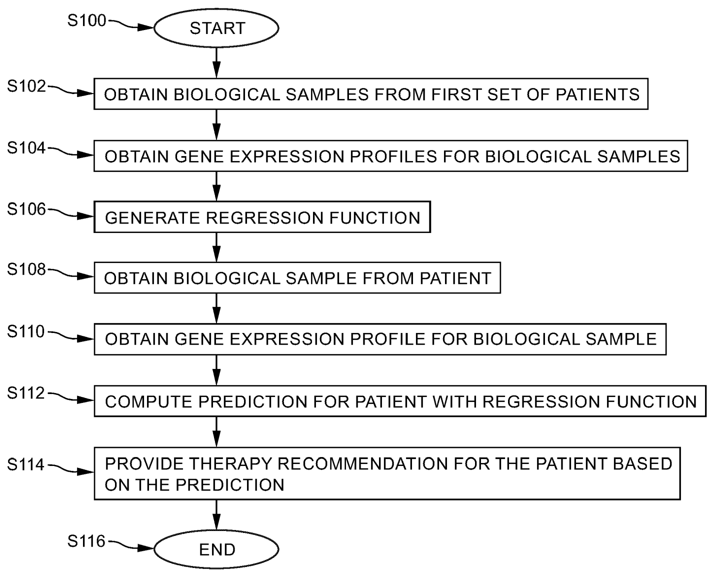


FIG. 1

EP 3 885 453 A1

SEQUENCE LISTING

<110> Koninklijke Philips N.V.

<120> Prediction of radiotherapy response for prostate cancer subject based on DNA repair genes

<130> 2020PF00060

<160> 24

<170> PatentIn version 3.5

<210> 1
<211> 2116
<212> DNA
<213> Homo sapiens

<400> 1
gacgtcatcc cgcagcgccg gaagcggtga ggcacagatg agtaacgtga atttgtccgt 60

ctccgacttc tggagattta tttttttag atgaggtgtt gctgtgttgc ccaagctgga 120

cttgaacccc taggctcaag caatccttcc acctcagtct cccaagtagc agggactaca 180

gagtgatgat gcgggtgtgc tggttggtga gacaggacag ccggcaccag cgaatcagac 240

ttccacattt ggaagcagtt gtgattgggc gtggcccaga gaccaagatc actgataaga 300

aatgttctcg acagcaagta cagttgaaag cagagtgtaa caagggatat gtcaaggtaa 360

agcaggtagg agtcaatccc accagcattg actcagtcgt aattgggaag gaccaagagg 420

tgaagctgca gcctggccag gttctccaca tggtgaatga actttatcca tatattgtag 480

agtttgagga agaggcaaag aaccctggcc tggaaacaca caggaagaga aagagatcag 540

gcaacagtga ttctatagaa agggatgctg ctcaggaagc tgaggctggg acagggctgg 600

aacctgggag caactctggc caatgctctg tgcccctaaa gaagggaaaa gatgcaccta 660

tcaaaaagga atccctgggc cactggagtc aaggcttgaa gatttctatg caggacccca 720

aaatgcaggt ttacaaagat gagcaggtgg tggtgataaa ggataaatac ccaaaggccc 780

gttaccattg gctggtctta ccgtggacct ccatttccag tctgaaggct gtggccaggg 840

aacaccttga actccttaag catatgcaca ctgtgggga aaaggtgatt gtagattttg 900

ctgggtccag caaactccgc ttccgattgg gctaccacgc cattccgagt atgagccatg 960

tacatcttca tgtgatcagc caggattttg attctccttg ccttaaaaac aaaaaacatt 1020

ggaattcttt caatacagaa tacttcctag aatcacaagc tgtgatcgag atggtacaag 1080

aggctggtag agtaactgtc cgagatggga tgcctgagct cttgaagctg ccccttcgtt 1140

gtcatgagtg ccagcagctg ctgccttcca ttcctcagct gaaagaacat ctcaggaagc 1200

actggacaca gtgattctgc agagcctgag ctgctgctgt ggtgtggccc actggagcaa 1260

actgctggca cctattctgg gttgcttgtg aacttctact catttcctaa attaaaacat 1320

```
gcagcttttt cacaaattta ttctattatt gagtggccac aatgtagagt ggctcaaagt    1380
acttcaggat taggaatttg ggtttgtcat agatgtattc tctggtgagg gtggctggga    1440
tatacctgac ccaccatctt cagaaggacc catgtcaggt ctgaccattg ggagcaaagc    1500
catgttcaca ctgacctaat gcagagtatg gaagcattgg gctggttata catttctgtt    1560
tcttagattt atcctccgcc tctgtaggca tggacaacct ttaatcagag catctagagt    1620
ggcctcttgt ttatcctgaa gatactgatg ggtcttgttt tctgttagtc tgttttgtaa    1680
tattcttttc ccttccttca tggggaggct tagtttgtcc agtccttcca tgcccttcta    1740
tcccagatta cctaaatgtt cccttctcag gaattctgtc tcatcagttc ttcacagtga    1800
gaaaagaggc tagatgatgg tgtgggggt tggagttttc ttctaatacc gagggttcct     1860
ggctgtgagg aaacagccac atgttcgtca tgattgagct gtgaagtctt cttggacctg    1920
ttgtctgaaa ataaagttaa tttgtttgag gcatctctct taagtaggtg gaaactattg    1980
aagttcagct aacaatcaca gcataggttc tgatgcatgg aaaggtggtt ggtgaatgaa    2040
aaagttgcgt agagccacta ctttcttttt ccctgagaat aaatttggat aaaacagttg    2100
tattcaaaaa aaaaaa                                                    2116
```

<210> 2
<211> 1870
<212> DNA
<213> Homo sapiens

<400> 2

```
aggcacagat gagtaacgtg aatttgtccg tctccgactt ctggaggtaa ggcggtcgtc      60
agcctatctc ttctgctggc tgggctcaat gccgcggagt gatgatgcgg gtgtgctggt     120
tggtgagaca ggacagccgg caccagcgaa tcagacttcc acatttggaa gcagttgtga     180
ttgggcgtgg cccagagacc aagatcactg ataagaaatg ttctcgacag caagagtttg     240
aggaagaggc aaagaaccct ggcctggaaa cacacaggaa gagaaagaga tcaggcaaca     300
gtgattctat agaaagggat gctgctcagg aagctgaggc tgggacaggg ctggaacctg     360
ggagcaactc tggccaatgc tctgtgcccc taaagaaggg aaaagatgca cctatcaaaa     420
aggaatccct gggccactgg agtcaaggct tgaagatttc tatgcaggac cccaaaatgc     480
aggtttacaa agatgagcag gtggtggtga taaaggataa atacccaaag gcccgttacc     540
attggctggt cttaccgtgg acctccattt ccagtctgaa ggctgtggcc agggaacacc     600
ttgaactcct taagcatatg cacactgtgg gggaaaaggt gattgtagat tttgctgggt     660
ccagcaaact ccgcttccga ttgggctacc acgccattcc gagtatgagc catgtacatc     720
ttcatgtgat cagccaggat tttgattctc cttgccttaa aaacaaaaaa cattggaatt     780
ctttcaatac agaatacttc ctagaatcac aagctgtgat cgagatggta caagaggctg     840
```

gtagagtaac tgtccgagat gggatgcctg agctcttgaa gctgcccctt cgttgtcatg 900

agtgccagca gctgctgcct tccattcctc agctgaaaga acatctcagg aagcactgga 960

cacagtgatt ctgcagagcc tgagctgctg ctgtggtgtg gcccactgga gcaaactgct 1020

ggcacctatt ctgggttgct tgtgaacttc tactcatttc ctaaattaaa acatgcagct 1080

ttttcacaaa tttattctat tattgagtgg ccacaatgta gagtggctca aagtacttca 1140

ggattaggaa tttgggtttg tcatagatgt attctctggt gagggtggct gggatatacc 1200

tgacccacca tcttcagaag gacccatgtc aggtctgacc attgggagca aagccatgtt 1260

cacactgacc taatgcagag tatggaagca ttgggctggt tatacatttc tgtttcttag 1320

atttatcctc cgcctctgta ggcatggaca acctttaatc agagcatcta gagtggcctc 1380

ttgtttatcc tgaagatact gatgggtctt gttttctgtt agtctgtttt gtaatattct 1440

tttcccttcc ttcatgggga ggcttagttt gtccagtcct tccatgccct tctatcccag 1500

attacctaaa tgttcccttc tcaggaattc tgtctcatca gttcttcaca gtgagaaaag 1560

aggctagatg atggtgtggg gggttggagt tttcttctaa taccgagggt tcctggctgt 1620

gaggaaacag ccacatgttc gtcatgattg agctgtgaag tcttcttgga cctgttgtct 1680

gaaaataaag ttaatttgtt tgaggcatct ctcttaagta ggtggaaact attgaagttc 1740

agctaacaat cacagcatag gttctgatgc atggaaaggt ggttggtgaa tgaaaaagtt 1800

gcgtagagcc actactttct ttttccctga gaataaattt ggataaaaca gttgtattca 1860

aaaaaaaaaa 1870

<210> 3
<211> 2013
<212> DNA
<213> Homo sapiens

<400> 3
agcgccggaa gcggtgaggc acagatgagt aacgtgaatt tgtccgtctc cgacttctgg 60

agagtgatga tgcgggtgtg ctggttggtg agacaggaca gccggcacca gcgaatcaga 120

cttccacatt tggaagcagt tgtgattggg cgtggcccag agaccaagat cactgataag 180

aaatgttctc gacagcaagt acagttgaaa gcagagtgta acaagggata tgtcaaggta 240

aagcaggtag gagtcaatcc caccagcatt gactcagtcg taattgggaa ggaccaagag 300

gtgaagctgc agcctggcca ggttctccac atggtgaatg aactttatcc atatattgta 360

gagtttgagg aagaggcaaa gaaccctggc ctggaaacac acaggaagag aaagagatca 420

ggcaacagtg attctataga aagggatgct gctcaggaag ctgaggctgg gacagggctg 480

gaacctggga gcaactctgg ccaatgctct gtgcccctaa agaagggaaa agatgcacct 540

atcaaaaagg aatccctggg ccactggagt caaggcttga agatttctat gcaggacccc 600

aaaatgcagg tttacaaaga tgagcaggtg gtggtgataa aggataaata cccaaaggcc 660

cgttaccatt ggctggtctt accgtggacc tccatttcca gtctgaaggc tgtggccagg 720

gaacaccttg aactccttaa gcatatgcac actgtggggg aaaaggtgat tgtagatttt 780

gctgggtcca gcaaactccg cttccgattg ggctaccacg ccattccgag tatgagccat 840

gtacatcttc atgtgatcag ccaggatttt gattctcctt gccttaaaaa caaaaaacat 900

tggaattctt tcaatacaga atacttccta gaatcacaag agtagctggg atttgacttt 960

tcttccttgc ctgcagctgt gatcgagatg gtacaagagg ctggtagagt aactgtccga 1020

gatgggatgc ctgagctctt gaagctgccc cttcgttgtc atgagtgcca gcagctgctg 1080

ccttccattc ctcagctgaa agaacatctc aggaagcact ggacacagtg attctgcaga 1140

gcctgagctg ctgctgtggt gtggcccact ggagcaaact gctggcacct attctgggtt 1200

gcttgtgaac ttctactcat ttcctaaatt aaaacatgca gctttttcac aaatttattc 1260

tattattgag tggccacaat gtagagtggc tcaaagtact tcaggattag gaatttgggt 1320

ttgtcataga tgtattctct ggtgagggtg gctgggatat acctgaccca ccatcttcag 1380

aaggacccat gtcaggtctg accattggga gcaaagccat gttcacactg acctaatgca 1440

gagtatggaa gcattgggct ggttatacat ttctgtttct tagatttatc ctccgcctct 1500

gtaggcatgg acaaccttta atcagagcat ctagagtggc ctcttgttta tcctgaagat 1560

actgatgggt cttgttttct gttagtctgt tttgtaatat tcttttccct tccttcatgg 1620

ggaggcttag tttgtccagt ccttccatgc ccttctatcc cagattacct aaatgttccc 1680

ttctcaggaa ttctgtctca tcagttcttc acagtgagaa aagaggctag atgatggtgt 1740

gggggttgg agttttcttc taataccgag ggttcctggc tgtgaggaaa cagccacatg 1800

ttcgtcatga ttgagctgtg aagtcttctt ggacctgttg tctgaaaata aagttaattt 1860

gtttgaggca tctctcttaa gtaggtggaa actattgaag ttcagctaac aatcacagca 1920

taggttctga tgcatggaaa ggtggttggt gaatgaaaaa gttgcgtaga gccactactt 1980

tctttttccc tgagaataaa tttggataaa aca 2013

<210> 4
<211> 1815
<212> DNA
<213> Homo sapiens

<400> 4
agcgccggaa gcggtgaggc acagatgagt aacgtgaatt tgtccgtctc cgacttctgg 60

agagtgatga tgcgggtgtg ctggttggtg agacaggaca gccggcacca gcgaatcaga 120

cttccacatt tggaagcagt tgtgattggg cgtggcccag agaccaagat cactgataag 180

aaatgttctc gacagcaaga gtttgaggaa gaggcaaaga accctggcct ggaaacacac 240

aggaagagaa agagatcagg caacagtgat tctatagaaa gggatgctgc tcaggaagct 300

gaggctggga cagggctgga acctgggagc aactctggcc aatgctctgt gcccctaaag 360

aagggaaaag atgcacctat caaaaaggaa tccctgggcc actggagtca aggcttgaag 420

atttctatgc aggaccccaa aatgcaggtt tacaaagatg agcaggtggt ggtgataaag 480

gataaatacc caaaggcccg ttaccattgg ctggtcttac cgtggacctc catttccagt 540

ctgaaggctg tggccaggga acaccttgaa ctccttaagc atatgcacac tgtgggggaa 600

aaggtgattg tagattttgc tgggtccagc aaactccgct tccgattggg ctaccacgcc 660

attccgagta tgagccatgt acatcttcat gtgatcagcc aggattttga ttctccttgc 720

cttaaaaaca aaaaacattg gaattctttc aatacagaat acttcctaga atcacaagct 780

gtgatcgaga tggtacaaga ggctggtaga gtaactgtcc gagatgggat gcctgagctc 840

ttgaagctgc cccttcgttg tcatgagtgc cagcagctgc tgccttccat tcctcagctg 900

aaagaacatc tcaggaagca ctggacacag tgattctgca gagcctgagc tgctgctgtg 960

gtgtggccca ctggagcaaa ctgctggcac ctattctggg ttgcttgtga acttctactc 1020

atttcctaaa ttaaaacatg cagctttttc acaaatttat tctattattg agtggccaca 1080

atgtagagtg gctcaaagta cttcaggatt aggaatttgg gtttgtcata gatgtattct 1140

ctggtgaggg tggctgggat atacctgacc caccatcttc agaaggaccc atgtcaggtc 1200

tgaccattgg gagcaaagcc atgttcacac tgacctaatg cagagtatgg aagcattggg 1260

ctggttatac atttctgttt cttagattta tcctccgcct ctgtaggcat ggacaacctt 1320

taatcagagc atctagagtg gcctcttgtt tatcctgaag atactgatgg gtcttgtttt 1380

ctgttagtct gttttgtaat attcttttcc cttccttcat ggggaggctt agtttgtcca 1440

gtccttccat gcccttctat cccagattac ctaaatgttc ccttctcagg aattctgtct 1500

catcagttct tcacagtgag aaaagaggct agatgatggt gtgggggtt ggagttttct 1560

tctaataccg agggttcctg gctgtgagga aacagccaca tgttcgtcat gattgagctg 1620

tgaagtcttc ttggacctgt tgtctgaaaa taaagttaat ttgtttgagg catctctctt 1680

aagtaggtgg aaactattga agttcagcta acaatcacag cataggttct gatgcatgga 1740

aaggtggttg gtgaatgaaa aagttgcgta gagccactac tttctttttc cctgagaata 1800

aatttggata aaaca 1815

<210> 5
<211> 342
<212> PRT
<213> Homo sapiens

<400> 5

Met Met Arg Val Cys Trp Leu Val Arg Gln Asp Ser Arg His Gln Arg

```
1               5                   10                  15

Ile Arg Leu Pro His Leu Glu Ala Val Val Ile Gly Arg Gly Pro Glu
            20                  25                  30

Thr Lys Ile Thr Asp Lys Lys Cys Ser Arg Gln Gln Val Gln Leu Lys
        35                  40                  45

Ala Glu Cys Asn Lys Gly Tyr Val Lys Val Lys Gln Val Gly Val Asn
    50                  55                  60

Pro Thr Ser Ile Asp Ser Val Val Ile Gly Lys Asp Gln Glu Val Lys
65                  70                  75                  80

Leu Gln Pro Gly Gln Val Leu His Met Val Asn Glu Leu Tyr Pro Tyr
                85                  90                  95

Ile Val Glu Phe Glu Glu Glu Ala Lys Asn Pro Gly Leu Glu Thr His
            100                 105                 110

Arg Lys Arg Lys Arg Ser Gly Asn Ser Asp Ser Ile Glu Arg Asp Ala
        115                 120                 125

Ala Gln Glu Ala Glu Ala Gly Thr Gly Leu Glu Pro Gly Ser Asn Ser
    130                 135                 140

Gly Gln Cys Ser Val Pro Leu Lys Lys Gly Lys Asp Ala Pro Ile Lys
145                 150                 155                 160

Lys Glu Ser Leu Gly His Trp Ser Gln Gly Leu Lys Ile Ser Met Gln
                165                 170                 175

Asp Pro Lys Met Gln Val Tyr Lys Asp Glu Gln Val Val Val Ile Lys
            180                 185                 190

Asp Lys Tyr Pro Lys Ala Arg Tyr His Trp Leu Val Leu Pro Trp Thr
        195                 200                 205

Ser Ile Ser Ser Leu Lys Ala Val Ala Arg Glu His Leu Glu Leu Leu
    210                 215                 220

Lys His Met His Thr Val Gly Glu Lys Val Ile Val Asp Phe Ala Gly
225                 230                 235                 240

Ser Ser Lys Leu Arg Phe Arg Leu Gly Tyr His Ala Ile Pro Ser Met
                245                 250                 255
```

Ser His Val His Leu His Val Ile Ser Gln Asp Phe Asp Ser Pro Cys
260 265 270

Leu Lys Asn Lys Lys His Trp Asn Ser Phe Asn Thr Glu Tyr Phe Leu
275 280 285

Glu Ser Gln Ala Val Ile Glu Met Val Gln Glu Ala Gly Arg Val Thr
290 295 300

Val Arg Asp Gly Met Pro Glu Leu Leu Lys Leu Pro Leu Arg Cys His
305 310 315 320

Glu Cys Gln Gln Leu Leu Pro Ser Ile Pro Gln Leu Lys Glu His Leu
325 330 335

Arg Lys His Trp Thr Gln
340

<210> 6
<211> 288
<212> PRT
<213> Homo sapiens

<400> 6

Met Met Arg Val Cys Trp Leu Val Arg Gln Asp Ser Arg His Gln Arg
1 5 10 15

Ile Arg Leu Pro His Leu Glu Ala Val Val Ile Gly Arg Gly Pro Glu
20 25 30

Thr Lys Ile Thr Asp Lys Lys Cys Ser Arg Gln Gln Glu Phe Glu Glu
35 40 45

Glu Ala Lys Asn Pro Gly Leu Glu Thr His Arg Lys Arg Lys Arg Ser
50 55 60

Gly Asn Ser Asp Ser Ile Glu Arg Asp Ala Ala Gln Glu Ala Glu Ala
65 70 75 80

Gly Thr Gly Leu Glu Pro Gly Ser Asn Ser Gly Gln Cys Ser Val Pro
85 90 95

Leu Lys Lys Gly Lys Asp Ala Pro Ile Lys Lys Glu Ser Leu Gly His
100 105 110

Trp Ser Gln Gly Leu Lys Ile Ser Met Gln Asp Pro Lys Met Gln Val
115 120 125

Tyr Lys Asp Glu Gln Val Val Val Ile Lys Asp Lys Tyr Pro Lys Ala
    130                 135                 140

Arg Tyr His Trp Leu Val Leu Pro Trp Thr Ser Ile Ser Ser Leu Lys
145                 150                 155                 160

Ala Val Ala Arg Glu His Leu Glu Leu Leu Lys His Met His Thr Val
                165                 170                 175

Gly Glu Lys Val Ile Val Asp Phe Ala Gly Ser Ser Lys Leu Arg Phe
            180                 185                 190

Arg Leu Gly Tyr His Ala Ile Pro Ser Met Ser His Val His Leu His
        195                 200                 205

Val Ile Ser Gln Asp Phe Asp Ser Pro Cys Leu Lys Asn Lys Lys His
    210                 215                 220

Trp Asn Ser Phe Asn Thr Glu Tyr Phe Leu Glu Ser Gln Ala Val Ile
225                 230                 235                 240

Glu Met Val Gln Glu Ala Gly Arg Val Thr Val Arg Asp Gly Met Pro
                245                 250                 255

Glu Leu Leu Lys Leu Pro Leu Arg Cys His Glu Cys Gln Gln Leu Leu
            260                 265                 270

Pro Ser Ile Pro Gln Leu Lys Glu His Leu Arg Lys His Trp Thr Gln
        275                 280                 285

<210> 7
<211> 292
<212> PRT
<213> Homo sapiens

<400> 7

Met Met Arg Val Cys Trp Leu Val Arg Gln Asp Ser Arg His Gln Arg
1               5                   10                  15

Ile Arg Leu Pro His Leu Glu Ala Val Val Ile Gly Arg Gly Pro Glu
            20                  25                  30

Thr Lys Ile Thr Asp Lys Lys Cys Ser Arg Gln Gln Val Gln Leu Lys
        35                  40                  45

Ala Glu Cys Asn Lys Gly Tyr Val Lys Val Lys Gln Val Gly Val Asn
    50                  55                  60

Pro Thr Ser Ile Asp Ser Val Val Ile Gly Lys Asp Gln Glu Val Lys
65 70 75 80

Leu Gln Pro Gly Gln Val Leu His Met Val Asn Glu Leu Tyr Pro Tyr
85 90 95

Ile Val Glu Phe Glu Glu Glu Ala Lys Asn Pro Gly Leu Glu Thr His
100 105 110

Arg Lys Arg Lys Arg Ser Gly Asn Ser Asp Ser Ile Glu Arg Asp Ala
115 120 125

Ala Gln Glu Ala Glu Ala Gly Thr Gly Leu Glu Pro Gly Ser Asn Ser
130 135 140

Gly Gln Cys Ser Val Pro Leu Lys Lys Gly Lys Asp Ala Pro Ile Lys
145 150 155 160

Lys Glu Ser Leu Gly His Trp Ser Gln Gly Leu Lys Ile Ser Met Gln
165 170 175

Asp Pro Lys Met Gln Val Tyr Lys Asp Glu Gln Val Val Val Ile Lys
180 185 190

Asp Lys Tyr Pro Lys Ala Arg Tyr His Trp Leu Val Leu Pro Trp Thr
195 200 205

Ser Ile Ser Ser Leu Lys Ala Val Ala Arg Glu His Leu Glu Leu Leu
210 215 220

Lys His Met His Thr Val Gly Glu Lys Val Ile Val Asp Phe Ala Gly
225 230 235 240

Ser Ser Lys Leu Arg Phe Arg Leu Gly Tyr His Ala Ile Pro Ser Met
245 250 255

Ser His Val His Leu His Val Ile Ser Gln Asp Phe Asp Ser Pro Cys
260 265 270

Leu Lys Asn Lys Lys His Trp Asn Ser Phe Asn Thr Glu Tyr Phe Leu
275 280 285

Glu Ser Gln Glu
290

<210> 8
<211> 288
<212> PRT

<213> Homo sapiens

<400> 8

```
Met Met Arg Val Cys Trp Leu Val Arg Gln Asp Ser Arg His Gln Arg
1               5                   10                  15

Ile Arg Leu Pro His Leu Glu Ala Val Val Ile Gly Arg Gly Pro Glu
            20                  25                  30

Thr Lys Ile Thr Asp Lys Lys Cys Ser Arg Gln Gln Glu Phe Glu Glu
        35                  40                  45

Glu Ala Lys Asn Pro Gly Leu Glu Thr His Arg Lys Arg Lys Arg Ser
    50                  55                  60

Gly Asn Ser Asp Ser Ile Glu Arg Asp Ala Ala Gln Glu Ala Glu Ala
65                  70                  75                  80

Gly Thr Gly Leu Glu Pro Gly Ser Asn Ser Gly Gln Cys Ser Val Pro
                85                  90                  95

Leu Lys Lys Gly Lys Asp Ala Pro Ile Lys Lys Glu Ser Leu Gly His
            100                 105                 110

Trp Ser Gln Gly Leu Lys Ile Ser Met Gln Asp Pro Lys Met Gln Val
        115                 120                 125

Tyr Lys Asp Glu Gln Val Val Val Ile Lys Asp Lys Tyr Pro Lys Ala
    130                 135                 140

Arg Tyr His Trp Leu Val Leu Pro Trp Thr Ser Ile Ser Ser Leu Lys
145                 150                 155                 160

Ala Val Ala Arg Glu His Leu Glu Leu Leu Lys His Met His Thr Val
                165                 170                 175

Gly Glu Lys Val Ile Val Asp Phe Ala Gly Ser Ser Lys Leu Arg Phe
            180                 185                 190

Arg Leu Gly Tyr His Ala Ile Pro Ser Met Ser His Val His Leu His
        195                 200                 205

Val Ile Ser Gln Asp Phe Asp Ser Pro Cys Leu Lys Asn Lys Lys His
    210                 215                 220

Trp Asn Ser Phe Asn Thr Glu Tyr Phe Leu Glu Ser Gln Ala Val Ile
225                 230                 235                 240
```

Glu Met Val Gln Glu Ala Gly Arg Val Thr Val Arg Asp Gly Met Pro
245 250 255

Glu Leu Leu Lys Leu Pro Leu Arg Cys His Glu Cys Gln Gln Leu Leu
260 265 270

Pro Ser Ile Pro Gln Leu Lys Glu His Leu Arg Lys His Trp Thr Gln
275 280 285

<210> 9
<211> 11386
<212> DNA
<213> Homo sapiens

<400> 9
gtggcgcgag cttctgaaac taggcggcag aggcggagcc gctgtggcac tgctgcgcct 60

ctgctgcgcc tcgggtgtct tttgcggcgg tgggtcgccg ccgggagaag cgtgagggga 120

cagatttgtg accggcgcgg tttttgtcag cttactccgg ccaaaaaaga actgcacctc 180

tggagcggac ttatttacca agcattggag gaatatcgta ggtaaaaatg cctattggat 240

ccaaagagag gccaacattt tttgaaattt ttaagacacg ctgcaacaaa gcagatttag 300

gaccaataag tcttaattgg tttgaagaac tttcttcaga agctccaccc tataattctg 360

aacctgcaga agaatctgaa cataaaaaca acaattacga accaaaccta tttaaaactc 420

cacaaaggaa accatcttat aatcagctgg cttcaactcc aataatattc aaagagcaag 480

ggctgactct gccgctgtac caatctcctg taaaagaatt agataaattc aaattagact 540

taggaaggaa tgttcccaat agtagacata aaagtcttcg cacagtgaaa actaaaatgg 600

atcaagcaga tgatgtttcc tgtccacttc taaattcttg tcttagtgaa agtcctgttg 660

ttctacaatg tacacatgta acaccacaaa gagataagtc agtggtatgt gggagtttgt 720

ttcatacacc aaagtttgtg aagggtcgtc agacaccaaa acatatttct gaaagtctag 780

gagctgaggt ggatcctgat atgtcttggt caagttcttt agctacacca cccaccctta 840

gttctactgt gctcatagtc agaaatgaag aagcatctga aactgtattt cctcatgata 900

ctactgctaa tgtgaaaagc tatttttcca atcatgatga aagtctgaag aaaaatgata 960

gatttatcgc ttctgtgaca gacagtgaaa acacaaatca aagagaagct gcaagtcatg 1020

gatttggaaa aacatcaggg aattcattta aagtaaatag ctgcaaagac cacattggaa 1080

agtcaatgcc aaatgtccta gaagatgaag tatatgaaac agttgtagat acctctgaag 1140

aagatagttt ttcattatgt ttttctaaat gtagaacaaa aaatctacaa aaagtaagaa 1200

ctagcaagac taggaaaaaa attttccatg aagcaaacgc tgatgaatgt gaaaaatcta 1260

aaaaccaagt gaaagaaaaa tactcatttg tatctgaagt ggaaccaaat gatactgatc 1320

cattagattc aaatgtagca aatcagaagc cctttgagag tggaagtgac aaaatctcca 1380
aggaagttgt accgtctttg gcctgtgaat ggtctcaact aaccctttca ggtctaaatg 1440
gagcccagat ggagaaaata cccctattgc atatttcttc atgtgaccaa aatatttcag 1500
aaaaagacct attagacaca gagaacaaaa gaaagaaaga ttttcttact tcagagaatt 1560
ctttgccacg tatttctagc ctaccaaaat cagagaagcc attaaatgag gaaacagtgg 1620
taaataagag agatgaagag cagcatcttg aatctcatac agactgcatt cttgcagtaa 1680
agcaggcaat atctggaact tctccagtgg cttcttcatt tcagggtatc aaaaagtcta 1740
tattcagaat aagagaatca cctaaagaga ctttcaatgc aagtttttca ggtcatatga 1800
ctgatccaaa ctttaaaaaa gaaactgaag cctctgaaag tggactggaa atacatactg 1860
tttgctcaca gaaggaggac tccttatgtc caaatttaat tgataatgga agctggccag 1920
ccaccaccac acagaattct gtagctttga agaatgcagg tttaatatcc actttgaaaa 1980
agaaaacaaa taagtttatt tatgctatac atgatgaaac atcttataaa ggaaaaaaaa 2040
taccgaaaga ccaaaaatca gaactaatta actgttcagc ccagtttgaa gcaaatgctt 2100
ttgaagcacc acttacattt gcaaatgctg attcaggttt attgcattct tctgtgaaaa 2160
gaagctgttc acagaatgat tctgaagaac caactttgtc cttaactagc tcttttggga 2220
caattctgag gaaatgttct agaaatgaaa catgttctaa taatacagta atctctcagg 2280
atcttgatta taaagaagca aaatgtaata aggaaaaact acagttattt attaccccag 2340
aagctgattc tctgtcatgc ctgcaggaag gacagtgtga aaatgatcca aaaagcaaaa 2400
aagtttcaga tataaaagaa gaggtcttgg ctgcagcatg tcacccagta caacattcaa 2460
aagtggaata cagtgatact gactttcaat cccagaaaag tcttttatat gatcatgaaa 2520
atgccagcac tcttatttta actcctactt ccaaggatgt tctgtcaaac ctagtcatga 2580
tttctagagg caaagaatca tacaaaatgt cagacaagct caaaggtaac aattatgaat 2640
ctgatgttga attaaccaaa aatattccca tggaaaagaa tcaagatgta tgtgctttaa 2700
atgaaaatta taaaaacgtt gagctgttgc cacctgaaaa atacatgaga gtagcatcac 2760
cttcaagaaa ggtacaattc aaccaaaaca caaatctaag agtaatccaa aaaaatcaag 2820
aagaaactac ttcaatttca aaaataactg tcaatccaga ctctgaagaa cttttctcag 2880
acaatgagaa taattttgtc ttccaagtag ctaatgaaag gaataatctt gctttaggaa 2940
atactaagga acttcatgaa acagacttga cttgtgtaaa cgaacccatt ttcaagaact 3000
ctaccatggt tttatatgga gacacaggtg ataaacaagc aacccaagtg tcaattaaaa 3060
aagatttggt ttatgttctt gcagaggaga acaaaaatag tgtaaagcag catataaaaa 3120
tgactctagg tcaagattta aaatcggaca tctccttgaa tatagataaa ataccagaaa 3180
aaaataatga ttacatgaac aaatgggcag gactcttagg tccaatttca aatcacagtt 3240

ttggaggtag cttcagaaca gcttcaaata aggaaatcaa gctctctgaa cataacatta 3300
agaagagcaa aatgttcttc aaagatattg aagaacaata tcctactagt ttagcttgtg 3360
ttgaaattgt aaataccttg gcattagata atcaaaagaa actgagcaag cctcagtcaa 3420
ttaatactgt atctgcacat ttacagagta gtgtagttgt ttctgattgt aaaaatagtc 3480
atataacccc tcagatgtta ttttccaagc aggattttaa ttcaaaccat aatttaacac 3540
ctagccaaaa ggcagaaatt acagaacttt ctactatatt agaagaatca ggaagtcagt 3600
ttgaatttac tcagtttaga aaaccaagct acatattgca gaagagtaca tttgaagtgc 3660
ctgaaaacca gatgactatc ttaaagacca cttctgagga atgcagagat gctgatcttc 3720
atgtcataat gaatgcccca tcgattggtc aggtagacag cagcaagcaa tttgaaggta 3780
cagttgaaat taaacggaag tttgctggcc tgttgaaaaa tgactgtaac aaaagtgctt 3840
ctggttattt aacagatgaa aatgaagtgg ggtttagggg cttttattct gctcatggca 3900
caaaactgaa tgtttctact gaagctctgc aaaaagctgt gaaactgttt agtgatattg 3960
agaatattag tgaggaaact tctgcagagg tacatccaat aagtttatct tcaagtaaat 4020
gtcatgattc tgttgtttca atgtttaaga tagaaaatca taatgataaa actgtaagtg 4080
aaaaaaataa taaatgccaa ctgatattac aaaataatat tgaaatgact actggcactt 4140
ttgttgaaga aattactgaa aattacaaga gaaatactga aaatgaagat aacaaatata 4200
ctgctgccag tagaaattct cataacttag aatttgatgg cagtgattca agtaaaaatg 4260
atactgtttg tattcataaa gatgaaacgg acttgctatt tactgatcag cacaacatat 4320
gtcttaaatt atctggccag tttatgaagg agggaaacac tcagattaaa gaagatttgt 4380
cagatttaac tttttggaa gttgcgaaag ctcaagaagc atgtcatggt aatacttcaa 4440
ataaagaaca gttaactgct actaaaacgg agcaaaatat aaaagatttt gagacttctg 4500
atacattttt tcagactgca agtgggaaaa atattagtgt cgccaaagag tcatttaata 4560
aaattgtaaa tttctttgat cagaaaccag aagaattgca taacttttcc ttaaattctg 4620
aattacattc tgacataaga aagaacaaaa tggacattct aagttatgag gaaacagaca 4680
tagttaaaca caaaatactg aaagaaagtg tcccagttgg tactggaaat caactagtga 4740
ccttccaggg acaacccgaa cgtgatgaaa agatcaaaga acctactcta ttgggttttc 4800
atacagctag cgggaaaaaa gttaaaattg caaaggaatc tttggacaaa gtgaaaaacc 4860
tttttgatga aaaagagcaa ggtactagtg aaatcaccag ttttagccat caatgggcaa 4920
agaccctaaa gtacagagag gcctgtaaag accttgaatt agcatgtgag accattgaga 4980
tcacagctgc cccaaagtgt aaagaaatgc agaattctct caataatgat aaaaaccttg 5040
tttctattga gactgtggtg ccacctaagc tcttaagtga taatttatgt agacaaactg 5100

aaaatctcaa aacatcaaaa agtatctttt tgaaagttaa agtacatgaa aatgtagaaa 5160
aagaaacagc aaaaagtcct gcaacttgtt acacaaatca gtccccttat tcagtcattg 5220
aaaattcagc cttagctttt tacacaagtt gtagtagaaa aacttctgtg agtcagactt 5280
cattacttga agcaaaaaaa tggcttagag aaggaatatt tgatggtcaa ccagaaagaa 5340
taaatactgc agattatgta ggaaattatt tgtatgaaaa taattcaaac agtactatag 5400
ctgaaaatga caaaaatcat ctctccgaaa aacaagatac ttatttaagt aacagtagca 5460
tgtctaacag ctattcctac cattctgatg aggtatataa tgattcagga tatctctcaa 5520
aaaataaact tgattctggt attgagccag tattgaagaa tgttgaagat caaaaaaaca 5580
ctagtttttc caaagtaata tccaatgtaa aagatgcaaa tgcataccca caaactgtaa 5640
atgaagatat ttgcgttgag gaacttgtga ctagctcttc accctgcaaa aataaaaatg 5700
cagccattaa attgtccata tctaatagta ataattttga ggtagggcca cctgcattta 5760
ggatagccag tggtaaaatc gtttgtgttt cacatgaaac aattaaaaaa gtgaaagaca 5820
tatttacaga cagtttcagt aaagtaatta aggaaaacaa cgagaataaa tcaaaaattt 5880
gccaaacgaa aattatggca ggttgttacg aggcattgga tgattcagag gatattcttc 5940
ataactctct agataatgat gaatgtagca cgcattcaca taaggttttt gctgacattc 6000
agagtgaaga aattttacaa cataaccaaa atatgtctgg attggagaaa gtttctaaaa 6060
tatcaccttg tgatgttagt ttggaaactt cagatatatg taaatgtagt atagggaagc 6120
ttcataagtc agtctcatct gcaaatactt gtgggatttt tagcacagca agtggaaaat 6180
ctgtccaggt atcagatgct tcattacaaa acgcaagaca agtgttttct gaaatagaag 6240
atagtaccaa gcaagtcttt tccaaagtat tgtttaaaag taacgaacat tcagaccagc 6300
tcacaagaga agaaaatact gctatacgta ctccagaaca tttaatatcc caaaaaggct 6360
tttcatataa tgtggtaaat tcatctgctt tctctggatt tagtacagca agtggaaagc 6420
aagtttccat tttagaaagt tccttacaca aagttaaggg agtgttagag gaatttgatt 6480
taatcagaac tgagcatagt cttcactatt cacctacgtc tagacaaaat gtatcaaaaa 6540
tacttcctcg tgttgataag agaaacccag agcactgtgt aaactcagaa atggaaaaaa 6600
cctgcagtaa agaatttaaa ttatcaaata acttaaatgt tgaaggtggt tcttcagaaa 6660
ataatcactc tattaaagtt tctccatatc tctctcaatt tcaacaagac aaacaacagt 6720
tggtattagg aaccaaagtg tcacttgttg agaacattca tgttttggga aaagaacagg 6780
cttcacctaa aaacgtaaaa atggaaattg gtaaaactga aactttttct gatgttcctg 6840
tgaaaacaaa tatagaagtt tgttctactt actccaaaga ttcagaaaac tactttgaaa 6900
cagaagcagt agaaattgct aaagctttta tggaagatga tgaactgaca gattctaaac 6960
tgccaagtca tgccacacat tctcttttta catgtcccga aaatgaggaa atggttttgt 7020

caaattcaag aattggaaaa agaagaggag agccccttat cttagtggga gaaccctcaa 7080
tcaaaagaaa cttattaaat gaatttgaca ggataataga aaatcaagaa aaatccttaa 7140
aggcttcaaa aagcactcca gatggcacaa taaaagatcg aagattgttt atgcatcatg 7200
tttctttaga gccgattacc tgtgtaccct ttcgcacaac taaggaacgt caagagatac 7260
agaatccaaa ttttaccgca cctggtcaag aatttctgtc taaatctcat ttgtatgaac 7320
atctgacttt ggaaaaatct tcaagcaatt tagcagtttc aggacatcca ttttatcaag 7380
tttctgctac aagaaatgaa aaaatgagac acttgattac tacaggcaga ccaaccaaag 7440
tctttgttcc accttttaaa actaaatcac attttcacag agttgaacag tgtgttagga 7500
atattaactt ggaggaaaac agacaaaagc aaaacattga tggacatggc tctgatgata 7560
gtaaaaataa gattaatgac aatgagattc atcagtttaa caaaaacaac tccaatcaag 7620
cagcagctgt aactttcaca aagtgtgaag aagaaccttt agatttaatt acaagtcttc 7680
agaatgccag agatatacag gatatgcgaa ttaagaagaa acaaaggcaa cgcgtctttc 7740
cacagccagg cagtctgtat cttgcaaaaa catccactct gcctcgaatc tctctgaaag 7800
cagcagtagg aggccaagtt ccctctgcgt gttctcataa acagctgtat acgtatggcg 7860
tttctaaaca ttgcataaaa attaacagca aaaatgcaga gtcttttcag tttcacactg 7920
aagattattt tggtaaggaa agtttatgga ctggaaaagg aatacagttg gctgatggtg 7980
gatggctcat accctccaat gatggaaagg ctggaaaaga agaattttat agggctctgt 8040
gtgacactcc aggtgtggat ccaaagctta tttctagaat ttgggtttat aatcactata 8100
gatggatcat atggaaactg gcagctatgg aatgtgcctt tcctaaggaa tttgctaata 8160
gatgcctaag cccagaaagg gtgcttcttc aactaaaata cagatatgat acggaaattg 8220
atagaagcag aagatcggct ataaaaaaga taatggaaag ggatgacaca gctgcaaaaa 8280
cacttgttct ctgtgtttct gacataattt cattgagcgc aaatatatct gaaacttcta 8340
gcaataaaac tagtagtgca gatacccaaa aagtggccat tattgaactt acagatgggt 8400
ggtatgctgt taaggcccag ttagatcctc ccctcttagc tgtcttaaag aatggcagac 8460
tgacagttgg tcagaagatt attcttcatg gagcagaact ggtgggctct cctgatgcct 8520
gtacacctct tgaagcccca gaatctctta tgttaaagat ttctgctaac agtactcggc 8580
ctgctcgctg gtataccaaa cttggattct ttcctgaccc tagacctttt cctctgccct 8640
tatcatcgct tttcagtgat ggaggaaatg ttggttgtgt tgatgtaatt attcaaagag 8700
catacccctat acagtggatg gagaagacat catctggatt atacatattt cgcaatgaaa 8760
gagaggaaga aaaggaagca gcaaaatatg tggaggccca acaaaagaga ctagaagcct 8820
tattcactaa aattcaggag gaatttgaag aacatgaaga aaacacaaca aaaccatatt 8880

taccatcacg tgcactaaca agacagcaag ttcgtgcttt gcaagatggt gcagagcttt 8940
atgaagcagt gaagaatgca gcagacccag cttaccttga gggttatttc agtgaagagc 9000
agttaagagc cttgaataat cacaggcaaa tgttgaatga taagaaacaa gctcagatcc 9060
agttggaaat taggaaggcc atggaatctg ctgaacaaaa ggaacaaggt ttatcaaggg 9120
atgtcacaac cgtgtggaag ttgcgtattg taagctattc aaaaaaagaa aaagattcag 9180
ttatactgag tatttggcgt ccatcatcag atttatattc tctgttaaca gaaggaaaga 9240
gatacagaat ttatcatctt gcaacttcaa aatctaaaag taaatctgaa agagctaaca 9300
tacagttagc agcgacaaaa aaaactcagt atcaacaact accggtttca gatgaaattt 9360
tatttcagat ttaccagcca cgggagcccc ttcacttcag caaattttta gatccagact 9420
ttcagccatc ttgttctgag gtggacctaa taggatttgt cgtttctgtt gtgaaaaaaa 9480
caggacttgc ccctttcgtc tatttgtcag acgaatgtta caatttactg gcaataaagt 9540
tttggataga ccttaatgag gacattatta agcctcatat gttaattgct gcaagcaacc 9600
tccagtggcg accagaatcc aaatcaggcc ttcttacttt atttgctgga gatttttctg 9660
tgttttctgc tagtccaaaa gagggccact ttcaagagac attcaacaaa atgaaaaata 9720
ctgttgagaa tattgacata ctttgcaatg aagcagaaaa caagcttatg catatactgc 9780
atgcaaatga tcccaagtgg tccaccccaa ctaaagactg tacttcaggg ccgtacactg 9840
ctcaaatcat tcctggtaca ggaaacaagc ttctgatgtc ttctcctaat tgtgagatat 9900
attatcaaag tcctttatca ctttgtatgg ccaaaaggaa gtctgtttcc acacctgtct 9960
cagcccagat gacttcaaag tcttgtaaag gggagaaaga gattgatgac caaaagaact 10020
gcaaaaagag aagagccttg gatttcttga gtagactgcc tttacctcca cctgttagtc 10080
ccatttgtac atttgtttct ccggctgcac agaaggcatt tcagccacca aggagttgtg 10140
gcaccaaata cgaaacaccc ataaagaaaa aagaactgaa ttctcctcag atgactccat 10200
ttaaaaaatt caatgaaatt tctcttttgg aaagtaattc aatagctgac gaagaacttg 10260
cattgataaa tacccaagct cttttgtctg gttcaacagg agaaaaacaa tttatatctg 10320
tcagtgaatc cactaggact gctcccacca gttcagaaga ttatctcaga ctgaaacgac 10380
gttgtactac atctctgatc aaagaacagg agagttccca ggccagtacg gaagaatgtg 10440
agaaaaataa gcaggacaca attacaacta aaaaatatat ctaagcattt gcaaaggcga 10500
caataaatta ttgacgctta acctttccag tttataagac tggaatataa tttcaaacca 10560
cacattagta cttatgttgc acaatgagaa aagaaattag tttcaaattt acctcagcgt 10620
ttgtgtatcg ggcaaaaatc gttttgcccg attccgtatt ggtatacttt tgcttcagtt 10680
gcatatctta aaactaaatg taatttatta actaatcaag aaaaacatct ttggctgagc 10740
tcggtggctc atgcctgtaa tcccaacact ttgagaagct gaggtgggag gagtgcttga 10800

ggccaggagt tcaagaccag cctgggcaac atagggagac ccccatcttt acaaagaaaa 10860

aaaaaagggg aaaagaaaat cttttaaatc tttggatttg atcactacaa gtattatttt 10920

acaagtgaaa taaacatacc attttctttt agattgtgtc attaaatgga atgaggtctc 10980

ttagtacagt tattttgatg cagataattc cttttagttt agctactatt ttaggggatt 11040

ttttttagag gtaactcact atgaaatagt tctccttaat gcaaatatgt tggttctgct 11100

atagttccat cctgttcaaa agtcaggatg aatatgaaga gtggtgtttc cttttgagca 11160

attcttcatc cttaagtcag catgattata agaaaaatag aaccctcagt gtaactctaa 11220

ttccttttta ctattccagt gtgatctctg aaattaaatt acttcaacta aaaattcaaa 11280

tactttaaat cagaagattt catagttaat ttattttttt tttcaacaaa atggtcatcc 11340

aaactcaaac ttgagaaaat atcttgcttt caaattggca ctgatt 11386

<210> 10
<211> 3418
<212> PRT
<213> Homo sapiens

<400> 10

Met Pro Ile Gly Ser Lys Glu Arg Pro Thr Phe Phe Glu Ile Phe Lys
1 5 10 15

Thr Arg Cys Asn Lys Ala Asp Leu Gly Pro Ile Ser Leu Asn Trp Phe
20 25 30

Glu Glu Leu Ser Ser Glu Ala Pro Pro Tyr Asn Ser Glu Pro Ala Glu
35 40 45

Glu Ser Glu His Lys Asn Asn Asn Tyr Glu Pro Asn Leu Phe Lys Thr
50 55 60

Pro Gln Arg Lys Pro Ser Tyr Asn Gln Leu Ala Ser Thr Pro Ile Ile
65 70 75 80

Phe Lys Glu Gln Gly Leu Thr Leu Pro Leu Tyr Gln Ser Pro Val Lys
85 90 95

Glu Leu Asp Lys Phe Lys Leu Asp Leu Gly Arg Asn Val Pro Asn Ser
100 105 110

Arg His Lys Ser Leu Arg Thr Val Lys Thr Lys Met Asp Gln Ala Asp
115 120 125

Asp Val Ser Cys Pro Leu Leu Asn Ser Cys Leu Ser Glu Ser Pro Val
130 135 140

Val Leu Gln Cys Thr His Val Thr Pro Gln Arg Asp Lys Ser Val Val
145 150 155 160

Cys Gly Ser Leu Phe His Thr Pro Lys Phe Val Lys Gly Arg Gln Thr
165 170 175

Pro Lys His Ile Ser Glu Ser Leu Gly Ala Glu Val Asp Pro Asp Met
180 185 190

Ser Trp Ser Ser Ser Leu Ala Thr Pro Pro Thr Leu Ser Ser Thr Val
195 200 205

Leu Ile Val Arg Asn Glu Glu Ala Ser Glu Thr Val Phe Pro His Asp
210 215 220

Thr Thr Ala Asn Val Lys Ser Tyr Phe Ser Asn His Asp Glu Ser Leu
225 230 235 240

Lys Lys Asn Asp Arg Phe Ile Ala Ser Val Thr Asp Ser Glu Asn Thr
245 250 255

Asn Gln Arg Glu Ala Ala Ser His Gly Phe Gly Lys Thr Ser Gly Asn
260 265 270

Ser Phe Lys Val Asn Ser Cys Lys Asp His Ile Gly Lys Ser Met Pro
275 280 285

Asn Val Leu Glu Asp Glu Val Tyr Glu Thr Val Val Asp Thr Ser Glu
290 295 300

Glu Asp Ser Phe Ser Leu Cys Phe Ser Lys Cys Arg Thr Lys Asn Leu
305 310 315 320

Gln Lys Val Arg Thr Ser Lys Thr Arg Lys Lys Ile Phe His Glu Ala
325 330 335

Asn Ala Asp Glu Cys Glu Lys Ser Lys Asn Gln Val Lys Glu Lys Tyr
340 345 350

Ser Phe Val Ser Glu Val Glu Pro Asn Asp Thr Asp Pro Leu Asp Ser
355 360 365

Asn Val Ala Asn Gln Lys Pro Phe Glu Ser Gly Ser Asp Lys Ile Ser
370 375 380

Lys Glu Val Val Pro Ser Leu Ala Cys Glu Trp Ser Gln Leu Thr Leu

385 390 395 400

Ser Gly Leu Asn Gly Ala Gln Met Glu Lys Ile Pro Leu Leu His Ile
405 410 415

Ser Ser Cys Asp Gln Asn Ile Ser Glu Lys Asp Leu Leu Asp Thr Glu
420 425 430

Asn Lys Arg Lys Lys Asp Phe Leu Thr Ser Glu Asn Ser Leu Pro Arg
435 440 445

Ile Ser Ser Leu Pro Lys Ser Glu Lys Pro Leu Asn Glu Glu Thr Val
450 455 460

Val Asn Lys Arg Asp Glu Glu Gln His Leu Glu Ser His Thr Asp Cys
465 470 475 480

Ile Leu Ala Val Lys Gln Ala Ile Ser Gly Thr Ser Pro Val Ala Ser
485 490 495

Ser Phe Gln Gly Ile Lys Lys Ser Ile Phe Arg Ile Arg Glu Ser Pro
500 505 510

Lys Glu Thr Phe Asn Ala Ser Phe Ser Gly His Met Thr Asp Pro Asn
515 520 525

Phe Lys Lys Glu Thr Glu Ala Ser Glu Ser Gly Leu Glu Ile His Thr
530 535 540

Val Cys Ser Gln Lys Glu Asp Ser Leu Cys Pro Asn Leu Ile Asp Asn
545 550 555 560

Gly Ser Trp Pro Ala Thr Thr Thr Gln Asn Ser Val Ala Leu Lys Asn
565 570 575

Ala Gly Leu Ile Ser Thr Leu Lys Lys Lys Thr Asn Lys Phe Ile Tyr
580 585 590

Ala Ile His Asp Glu Thr Ser Tyr Lys Gly Lys Lys Ile Pro Lys Asp
595 600 605

Gln Lys Ser Glu Leu Ile Asn Cys Ser Ala Gln Phe Glu Ala Asn Ala
610 615 620

Phe Glu Ala Pro Leu Thr Phe Ala Asn Ala Asp Ser Gly Leu Leu His
625 630 635 640

Ser Ser Val Lys Arg Ser Cys Ser Gln Asn Asp Ser Glu Glu Pro Thr
645 650 655

Leu Ser Leu Thr Ser Ser Phe Gly Thr Ile Leu Arg Lys Cys Ser Arg
660 665 670

Asn Glu Thr Cys Ser Asn Asn Thr Val Ile Ser Gln Asp Leu Asp Tyr
675 680 685

Lys Glu Ala Lys Cys Asn Lys Glu Lys Leu Gln Leu Phe Ile Thr Pro
690 695 700

Glu Ala Asp Ser Leu Ser Cys Leu Gln Glu Gly Gln Cys Glu Asn Asp
705 710 715 720

Pro Lys Ser Lys Lys Val Ser Asp Ile Lys Glu Glu Val Leu Ala Ala
725 730 735

Ala Cys His Pro Val Gln His Ser Lys Val Glu Tyr Ser Asp Thr Asp
740 745 750

Phe Gln Ser Gln Lys Ser Leu Leu Tyr Asp His Glu Asn Ala Ser Thr
755 760 765

Leu Ile Leu Thr Pro Thr Ser Lys Asp Val Leu Ser Asn Leu Val Met
770 775 780

Ile Ser Arg Gly Lys Glu Ser Tyr Lys Met Ser Asp Lys Leu Lys Gly
785 790 795 800

Asn Asn Tyr Glu Ser Asp Val Glu Leu Thr Lys Asn Ile Pro Met Glu
805 810 815

Lys Asn Gln Asp Val Cys Ala Leu Asn Glu Asn Tyr Lys Asn Val Glu
820 825 830

Leu Leu Pro Pro Glu Lys Tyr Met Arg Val Ala Ser Pro Ser Arg Lys
835 840 845

Val Gln Phe Asn Gln Asn Thr Asn Leu Arg Val Ile Gln Lys Asn Gln
850 855 860

Glu Glu Thr Thr Ser Ile Ser Lys Ile Thr Val Asn Pro Asp Ser Glu
865 870 875 880

Glu Leu Phe Ser Asp Asn Glu Asn Asn Phe Val Phe Gln Val Ala Asn
885 890 895

```
Glu Arg Asn Asn Leu Ala Leu Gly Asn Thr Lys Glu Leu His Glu Thr
            900                 905                 910

Asp Leu Thr Cys Val Asn Glu Pro Ile Phe Lys Asn Ser Thr Met Val
        915                 920                 925

Leu Tyr Gly Asp Thr Gly Asp Lys Gln Ala Thr Gln Val Ser Ile Lys
    930                 935                 940

Lys Asp Leu Val Tyr Val Leu Ala Glu Glu Asn Lys Asn Ser Val Lys
945                 950                 955                 960

Gln His Ile Lys Met Thr Leu Gly Gln Asp Leu Lys Ser Asp Ile Ser
                965                 970                 975

Leu Asn Ile Asp Lys Ile Pro Glu Lys Asn Asn Asp Tyr Met Asn Lys
            980                 985                 990

Trp Ala Gly Leu Leu Gly Pro Ile  Ser Asn His Ser Phe  Gly Gly Ser
        995                 1000                1005

Phe Arg  Thr Ala Ser Asn Lys  Glu Ile Lys Leu Ser  Glu His Asn
    1010                1015                1020

Ile Lys  Lys Ser Lys Met Phe  Phe Lys Asp Ile Glu  Glu Gln Tyr
    1025                1030                1035

Pro Thr  Ser Leu Ala Cys Val  Glu Ile Val Asn Thr  Leu Ala Leu
    1040                1045                1050

Asp Asn  Gln Lys Lys Leu Ser  Lys Pro Gln Ser Ile  Asn Thr Val
    1055                1060                1065

Ser Ala  His Leu Gln Ser Ser  Val Val Val Ser Asp  Cys Lys Asn
    1070                1075                1080

Ser His  Ile Thr Pro Gln Met  Leu Phe Ser Lys Gln  Asp Phe Asn
    1085                1090                1095

Ser Asn  His Asn Leu Thr Pro  Ser Gln Lys Ala Glu  Ile Thr Glu
    1100                1105                1110

Leu Ser  Thr Ile Leu Glu Glu  Ser Gly Ser Gln Phe  Glu Phe Thr
    1115                1120                1125

Gln Phe  Arg Lys Pro Ser Tyr  Ile Leu Gln Lys Ser  Thr Phe Glu
    1130                1135                1140
```

Val Pro Glu Asn Gln Met Thr Ile Leu Lys Thr Thr Ser Glu Glu
1145 1150 1155

Cys Arg Asp Ala Asp Leu His Val Ile Met Asn Ala Pro Ser Ile
1160 1165 1170

Gly Gln Val Asp Ser Ser Lys Gln Phe Glu Gly Thr Val Glu Ile
1175 1180 1185

Lys Arg Lys Phe Ala Gly Leu Leu Lys Asn Asp Cys Asn Lys Ser
1190 1195 1200

Ala Ser Gly Tyr Leu Thr Asp Glu Asn Glu Val Gly Phe Arg Gly
1205 1210 1215

Phe Tyr Ser Ala His Gly Thr Lys Leu Asn Val Ser Thr Glu Ala
1220 1225 1230

Leu Gln Lys Ala Val Lys Leu Phe Ser Asp Ile Glu Asn Ile Ser
1235 1240 1245

Glu Glu Thr Ser Ala Glu Val His Pro Ile Ser Leu Ser Ser Ser
1250 1255 1260

Lys Cys His Asp Ser Val Val Ser Met Phe Lys Ile Glu Asn His
1265 1270 1275

Asn Asp Lys Thr Val Ser Glu Lys Asn Asn Lys Cys Gln Leu Ile
1280 1285 1290

Leu Gln Asn Asn Ile Glu Met Thr Thr Gly Thr Phe Val Glu Glu
1295 1300 1305

Ile Thr Glu Asn Tyr Lys Arg Asn Thr Glu Asn Glu Asp Asn Lys
1310 1315 1320

Tyr Thr Ala Ala Ser Arg Asn Ser His Asn Leu Glu Phe Asp Gly
1325 1330 1335

Ser Asp Ser Ser Lys Asn Asp Thr Val Cys Ile His Lys Asp Glu
1340 1345 1350

Thr Asp Leu Leu Phe Thr Asp Gln His Asn Ile Cys Leu Lys Leu
1355 1360 1365

Ser Gly Gln Phe Met Lys Glu Gly Asn Thr Gln Ile Lys Glu Asp

1370 1375 1380

Leu Ser Asp Leu Thr Phe Leu Glu Val Ala Lys Ala Gln Glu Ala
1385 1390 1395

Cys His Gly Asn Thr Ser Asn Lys Glu Gln Leu Thr Ala Thr Lys
1400 1405 1410

Thr Glu Gln Asn Ile Lys Asp Phe Glu Thr Ser Asp Thr Phe Phe
1415 1420 1425

Gln Thr Ala Ser Gly Lys Asn Ile Ser Val Ala Lys Glu Ser Phe
1430 1435 1440

Asn Lys Ile Val Asn Phe Phe Asp Gln Lys Pro Glu Glu Leu His
1445 1450 1455

Asn Phe Ser Leu Asn Ser Glu Leu His Ser Asp Ile Arg Lys Asn
1460 1465 1470

Lys Met Asp Ile Leu Ser Tyr Glu Glu Thr Asp Ile Val Lys His
1475 1480 1485

Lys Ile Leu Lys Glu Ser Val Pro Val Gly Thr Gly Asn Gln Leu
1490 1495 1500

Val Thr Phe Gln Gly Gln Pro Glu Arg Asp Glu Lys Ile Lys Glu
1505 1510 1515

Pro Thr Leu Leu Gly Phe His Thr Ala Ser Gly Lys Lys Val Lys
1520 1525 1530

Ile Ala Lys Glu Ser Leu Asp Lys Val Lys Asn Leu Phe Asp Glu
1535 1540 1545

Lys Glu Gln Gly Thr Ser Glu Ile Thr Ser Phe Ser His Gln Trp
1550 1555 1560

Ala Lys Thr Leu Lys Tyr Arg Glu Ala Cys Lys Asp Leu Glu Leu
1565 1570 1575

Ala Cys Glu Thr Ile Glu Ile Thr Ala Ala Pro Lys Cys Lys Glu
1580 1585 1590

Met Gln Asn Ser Leu Asn Asn Asp Lys Asn Leu Val Ser Ile Glu
1595 1600 1605

Thr Val Val Pro Pro Lys Leu Leu Ser Asp Asn Leu Cys Arg Gln
1610 1615 1620

Thr Glu Asn Leu Lys Thr Ser Lys Ser Ile Phe Leu Lys Val Lys
1625 1630 1635

Val His Glu Asn Val Glu Lys Glu Thr Ala Lys Ser Pro Ala Thr
1640 1645 1650

Cys Tyr Thr Asn Gln Ser Pro Tyr Ser Val Ile Glu Asn Ser Ala
1655 1660 1665

Leu Ala Phe Tyr Thr Ser Cys Ser Arg Lys Thr Ser Val Ser Gln
1670 1675 1680

Thr Ser Leu Leu Glu Ala Lys Lys Trp Leu Arg Glu Gly Ile Phe
1685 1690 1695

Asp Gly Gln Pro Glu Arg Ile Asn Thr Ala Asp Tyr Val Gly Asn
1700 1705 1710

Tyr Leu Tyr Glu Asn Asn Ser Asn Ser Thr Ile Ala Glu Asn Asp
1715 1720 1725

Lys Asn His Leu Ser Glu Lys Gln Asp Thr Tyr Leu Ser Asn Ser
1730 1735 1740

Ser Met Ser Asn Ser Tyr Ser Tyr His Ser Asp Glu Val Tyr Asn
1745 1750 1755

Asp Ser Gly Tyr Leu Ser Lys Asn Lys Leu Asp Ser Gly Ile Glu
1760 1765 1770

Pro Val Leu Lys Asn Val Glu Asp Gln Lys Asn Thr Ser Phe Ser
1775 1780 1785

Lys Val Ile Ser Asn Val Lys Asp Ala Asn Ala Tyr Pro Gln Thr
1790 1795 1800

Val Asn Glu Asp Ile Cys Val Glu Glu Leu Val Thr Ser Ser Ser
1805 1810 1815

Pro Cys Lys Asn Lys Asn Ala Ala Ile Lys Leu Ser Ile Ser Asn
1820 1825 1830

Ser Asn Asn Phe Glu Val Gly Pro Pro Ala Phe Arg Ile Ala Ser
1835 1840 1845

Gly Lys Ile Val Cys Val Ser His Glu Thr Ile Lys Lys Val Lys
1850 1855 1860

Asp Ile Phe Thr Asp Ser Phe Ser Lys Val Ile Lys Glu Asn Asn
1865 1870 1875

Glu Asn Lys Ser Lys Ile Cys Gln Thr Lys Ile Met Ala Gly Cys
1880 1885 1890

Tyr Glu Ala Leu Asp Asp Ser Glu Asp Ile Leu His Asn Ser Leu
1895 1900 1905

Asp Asn Asp Glu Cys Ser Thr His Ser His Lys Val Phe Ala Asp
1910 1915 1920

Ile Gln Ser Glu Glu Ile Leu Gln His Asn Gln Asn Met Ser Gly
1925 1930 1935

Leu Glu Lys Val Ser Lys Ile Ser Pro Cys Asp Val Ser Leu Glu
1940 1945 1950

Thr Ser Asp Ile Cys Lys Cys Ser Ile Gly Lys Leu His Lys Ser
1955 1960 1965

Val Ser Ser Ala Asn Thr Cys Gly Ile Phe Ser Thr Ala Ser Gly
1970 1975 1980

Lys Ser Val Gln Val Ser Asp Ala Ser Leu Gln Asn Ala Arg Gln
1985 1990 1995

Val Phe Ser Glu Ile Glu Asp Ser Thr Lys Gln Val Phe Ser Lys
2000 2005 2010

Val Leu Phe Lys Ser Asn Glu His Ser Asp Gln Leu Thr Arg Glu
2015 2020 2025

Glu Asn Thr Ala Ile Arg Thr Pro Glu His Leu Ile Ser Gln Lys
2030 2035 2040

Gly Phe Ser Tyr Asn Val Val Asn Ser Ser Ala Phe Ser Gly Phe
2045 2050 2055

Ser Thr Ala Ser Gly Lys Gln Val Ser Ile Leu Glu Ser Ser Leu
2060 2065 2070

His Lys Val Lys Gly Val Leu Glu Glu Phe Asp Leu Ile Arg Thr
2075 2080 2085

Glu His Ser Leu His Tyr Ser Pro Thr Ser Arg Gln Asn Val Ser
2090 2095 2100

Lys Ile Leu Pro Arg Val Asp Lys Arg Asn Pro Glu His Cys Val
2105 2110 2115

Asn Ser Glu Met Glu Lys Thr Cys Ser Lys Glu Phe Lys Leu Ser
2120 2125 2130

Asn Asn Leu Asn Val Glu Gly Gly Ser Ser Glu Asn Asn His Ser
2135 2140 2145

Ile Lys Val Ser Pro Tyr Leu Ser Gln Phe Gln Gln Asp Lys Gln
2150 2155 2160

Gln Leu Val Leu Gly Thr Lys Val Ser Leu Val Glu Asn Ile His
2165 2170 2175

Val Leu Gly Lys Glu Gln Ala Ser Pro Lys Asn Val Lys Met Glu
2180 2185 2190

Ile Gly Lys Thr Glu Thr Phe Ser Asp Val Pro Val Lys Thr Asn
2195 2200 2205

Ile Glu Val Cys Ser Thr Tyr Ser Lys Asp Ser Glu Asn Tyr Phe
2210 2215 2220

Glu Thr Glu Ala Val Glu Ile Ala Lys Ala Phe Met Glu Asp Asp
2225 2230 2235

Glu Leu Thr Asp Ser Lys Leu Pro Ser His Ala Thr His Ser Leu
2240 2245 2250

Phe Thr Cys Pro Glu Asn Glu Glu Met Val Leu Ser Asn Ser Arg
2255 2260 2265

Ile Gly Lys Arg Arg Gly Glu Pro Leu Ile Leu Val Gly Glu Pro
2270 2275 2280

Ser Ile Lys Arg Asn Leu Leu Asn Glu Phe Asp Arg Ile Ile Glu
2285 2290 2295

Asn Gln Glu Lys Ser Leu Lys Ala Ser Lys Ser Thr Pro Asp Gly
2300 2305 2310

Thr Ile Lys Asp Arg Arg Leu Phe Met His His Val Ser Leu Glu

2315 2320 2325

Pro Ile Thr Cys Val Pro Phe Arg Thr Thr Lys Glu Arg Gln Glu
2330 2335 2340

Ile Gln Asn Pro Asn Phe Thr Ala Pro Gly Gln Glu Phe Leu Ser
2345 2350 2355

Lys Ser His Leu Tyr Glu His Leu Thr Leu Glu Lys Ser Ser Ser
2360 2365 2370

Asn Leu Ala Val Ser Gly His Pro Phe Tyr Gln Val Ser Ala Thr
2375 2380 2385

Arg Asn Glu Lys Met Arg His Leu Ile Thr Thr Gly Arg Pro Thr
2390 2395 2400

Lys Val Phe Val Pro Pro Phe Lys Thr Lys Ser His Phe His Arg
2405 2410 2415

Val Glu Gln Cys Val Arg Asn Ile Asn Leu Glu Glu Asn Arg Gln
2420 2425 2430

Lys Gln Asn Ile Asp Gly His Gly Ser Asp Asp Ser Lys Asn Lys
2435 2440 2445

Ile Asn Asp Asn Glu Ile His Gln Phe Asn Lys Asn Asn Ser Asn
2450 2455 2460

Gln Ala Ala Ala Val Thr Phe Thr Lys Cys Glu Glu Glu Pro Leu
2465 2470 2475

Asp Leu Ile Thr Ser Leu Gln Asn Ala Arg Asp Ile Gln Asp Met
2480 2485 2490

Arg Ile Lys Lys Lys Gln Arg Gln Arg Val Phe Pro Gln Pro Gly
2495 2500 2505

Ser Leu Tyr Leu Ala Lys Thr Ser Thr Leu Pro Arg Ile Ser Leu
2510 2515 2520

Lys Ala Ala Val Gly Gly Gln Val Pro Ser Ala Cys Ser His Lys
2525 2530 2535

Gln Leu Tyr Thr Tyr Gly Val Ser Lys His Cys Ile Lys Ile Asn
2540 2545 2550

Ser Lys Asn Ala Glu Ser Phe Gln Phe His Thr Glu Asp Tyr Phe
2555 2560 2565

Gly Lys Glu Ser Leu Trp Thr Gly Lys Gly Ile Gln Leu Ala Asp
2570 2575 2580

Gly Gly Trp Leu Ile Pro Ser Asn Asp Gly Lys Ala Gly Lys Glu
2585 2590 2595

Glu Phe Tyr Arg Ala Leu Cys Asp Thr Pro Gly Val Asp Pro Lys
2600 2605 2610

Leu Ile Ser Arg Ile Trp Val Tyr Asn His Tyr Arg Trp Ile Ile
2615 2620 2625

Trp Lys Leu Ala Ala Met Glu Cys Ala Phe Pro Lys Glu Phe Ala
2630 2635 2640

Asn Arg Cys Leu Ser Pro Glu Arg Val Leu Leu Gln Leu Lys Tyr
2645 2650 2655

Arg Tyr Asp Thr Glu Ile Asp Arg Ser Arg Arg Ser Ala Ile Lys
2660 2665 2670

Lys Ile Met Glu Arg Asp Asp Thr Ala Ala Lys Thr Leu Val Leu
2675 2680 2685

Cys Val Ser Asp Ile Ile Ser Leu Ser Ala Asn Ile Ser Glu Thr
2690 2695 2700

Ser Ser Asn Lys Thr Ser Ser Ala Asp Thr Gln Lys Val Ala Ile
2705 2710 2715

Ile Glu Leu Thr Asp Gly Trp Tyr Ala Val Lys Ala Gln Leu Asp
2720 2725 2730

Pro Pro Leu Leu Ala Val Leu Lys Asn Gly Arg Leu Thr Val Gly
2735 2740 2745

Gln Lys Ile Ile Leu His Gly Ala Glu Leu Val Gly Ser Pro Asp
2750 2755 2760

Ala Cys Thr Pro Leu Glu Ala Pro Glu Ser Leu Met Leu Lys Ile
2765 2770 2775

Ser Ala Asn Ser Thr Arg Pro Ala Arg Trp Tyr Thr Lys Leu Gly
2780 2785 2790

Phe Phe Pro Asp Pro Arg Pro Phe Pro Leu Pro Leu Ser Ser Leu
2795 2800 2805

Phe Ser Asp Gly Gly Asn Val Gly Cys Val Asp Val Ile Ile Gln
2810 2815 2820

Arg Ala Tyr Pro Ile Gln Trp Met Glu Lys Thr Ser Ser Gly Leu
2825 2830 2835

Tyr Ile Phe Arg Asn Glu Arg Glu Glu Glu Lys Glu Ala Ala Lys
2840 2845 2850

Tyr Val Glu Ala Gln Gln Lys Arg Leu Glu Ala Leu Phe Thr Lys
2855 2860 2865

Ile Gln Glu Glu Phe Glu Glu His Glu Glu Asn Thr Thr Lys Pro
2870 2875 2880

Tyr Leu Pro Ser Arg Ala Leu Thr Arg Gln Gln Val Arg Ala Leu
2885 2890 2895

Gln Asp Gly Ala Glu Leu Tyr Glu Ala Val Lys Asn Ala Ala Asp
2900 2905 2910

Pro Ala Tyr Leu Glu Gly Tyr Phe Ser Glu Glu Gln Leu Arg Ala
2915 2920 2925

Leu Asn Asn His Arg Gln Met Leu Asn Asp Lys Lys Gln Ala Gln
2930 2935 2940

Ile Gln Leu Glu Ile Arg Lys Ala Met Glu Ser Ala Glu Gln Lys
2945 2950 2955

Glu Gln Gly Leu Ser Arg Asp Val Thr Thr Val Trp Lys Leu Arg
2960 2965 2970

Ile Val Ser Tyr Ser Lys Lys Glu Lys Asp Ser Val Ile Leu Ser
2975 2980 2985

Ile Trp Arg Pro Ser Ser Asp Leu Tyr Ser Leu Leu Thr Glu Gly
2990 2995 3000

Lys Arg Tyr Arg Ile Tyr His Leu Ala Thr Ser Lys Ser Lys Ser
3005 3010 3015

Lys Ser Glu Arg Ala Asn Ile Gln Leu Ala Ala Thr Lys Lys Thr
3020 3025 3030

Gln Tyr Gln Gln Leu Pro Val Ser Asp Glu Ile Leu Phe Gln Ile
3035 3040 3045

Tyr Gln Pro Arg Glu Pro Leu His Phe Ser Lys Phe Leu Asp Pro
3050 3055 3060

Asp Phe Gln Pro Ser Cys Ser Glu Val Asp Leu Ile Gly Phe Val
3065 3070 3075

Val Ser Val Val Lys Lys Thr Gly Leu Ala Pro Phe Val Tyr Leu
3080 3085 3090

Ser Asp Glu Cys Tyr Asn Leu Leu Ala Ile Lys Phe Trp Ile Asp
3095 3100 3105

Leu Asn Glu Asp Ile Ile Lys Pro His Met Leu Ile Ala Ala Ser
3110 3115 3120

Asn Leu Gln Trp Arg Pro Glu Ser Lys Ser Gly Leu Leu Thr Leu
3125 3130 3135

Phe Ala Gly Asp Phe Ser Val Phe Ser Ala Ser Pro Lys Glu Gly
3140 3145 3150

His Phe Gln Glu Thr Phe Asn Lys Met Lys Asn Thr Val Glu Asn
3155 3160 3165

Ile Asp Ile Leu Cys Asn Glu Ala Glu Asn Lys Leu Met His Ile
3170 3175 3180

Leu His Ala Asn Asp Pro Lys Trp Ser Thr Pro Thr Lys Asp Cys
3185 3190 3195

Thr Ser Gly Pro Tyr Thr Ala Gln Ile Ile Pro Gly Thr Gly Asn
3200 3205 3210

Lys Leu Leu Met Ser Ser Pro Asn Cys Glu Ile Tyr Tyr Gln Ser
3215 3220 3225

Pro Leu Ser Leu Cys Met Ala Lys Arg Lys Ser Val Ser Thr Pro
3230 3235 3240

Val Ser Ala Gln Met Thr Ser Lys Ser Cys Lys Gly Glu Lys Glu
3245 3250 3255

Ile Asp Asp Gln Lys Asn Cys Lys Lys Arg Arg Ala Leu Asp Phe

3260 3265 3270

Leu Ser Arg Leu Pro Leu Pro Pro Pro Val Ser Pro Ile Cys Thr
3275 3280 3285

Phe Val Ser Pro Ala Ala Gln Lys Ala Phe Gln Pro Pro Arg Ser
3290 3295 3300

Cys Gly Thr Lys Tyr Glu Thr Pro Ile Lys Lys Lys Glu Leu Asn
3305 3310 3315

Ser Pro Gln Met Thr Pro Phe Lys Lys Phe Asn Glu Ile Ser Leu
3320 3325 3330

Leu Glu Ser Asn Ser Ile Ala Asp Glu Glu Leu Ala Leu Ile Asn
3335 3340 3345

Thr Gln Ala Leu Leu Ser Gly Ser Thr Gly Glu Lys Gln Phe Ile
3350 3355 3360

Ser Val Ser Glu Ser Thr Arg Thr Ala Pro Thr Ser Ser Glu Asp
3365 3370 3375

Tyr Leu Arg Leu Lys Arg Arg Cys Thr Thr Ser Leu Ile Lys Glu
3380 3385 3390

Gln Glu Ser Ser Gln Ala Ser Thr Glu Glu Cys Glu Lys Asn Lys
3395 3400 3405

Gln Asp Thr Ile Thr Thr Lys Lys Tyr Ile
3410 3415

<210> 11
<211> 801
<212> DNA
<213> Homo sapiens

<400> 11
agggctttct gtatccctag gtttcttgcc ttgatgtact ggagcaatca gatcacacgg 60

cggcttggag agtgagtgca aggttttatg agtggaatta gccctcagca gatgggggag 120

ccagaaggca gttggagtgg gaagaaccca gggaccatgg gcgcctccag gctctatacc 180

ctggtgctgg tcctgcagcc tcagcgagtt ctcctgggca tgaaaaagcg aggcttcggg 240

gccggccggt ggaatggctt tgggggcaaa gtgcaagaag gagagaccat cgaggatggg 300

gctaggaggg agctgcagga ggagagcggt ctgacagtgg acgccctgca caaggtgggc 360

cagatcgtgt ttgagttcgt gggcgagcct gagctcatgg acgtgcatgt cttctgcaca 420

gacagcatcc aggggacccc cgtggagagc gacgaaatgc gcccatgctg gttccagctg 480

gatcagatcc ccttcaagga catgtggccc gacgacagct actggtttcc actcctgctt 540

cagaagaaga aattccacgg gtacttcaag ttccagggtc aggacaccat cctggactac 600

acactccgcg aggtggacac ggtctagcgg gagcccaggg cagcccctgg gcaggagacg 660

tggctgctga acagccgcaa accatcttca cctgggggca ttgagtggcg cagagccggg 720

tttcatctgg aattaactgg atggaaggga aaataaagct atctagcggt gaaaaaaaaa 780

aaaaaaaaaa aaaaaaaaaa a 801

<210> 12
<211> 728
<212> DNA
<213> Homo sapiens

<400> 12
agggctttct gtatccctag gtttcttgcc ttgatgtact ggagcaatca gatcacacgg 60

cggcttggag aaacccaggg accatgggcg cctccaggct ctataccctg gtgctggtcc 120

tgcagcctca gcgagttctc ctgggcatga aaaagcgagg cttcggggcc ggccggtgga 180

atggctttgg gggcaaagtg caagaaggag agaccatcga ggatggggct aggagggagc 240

tgcaggagga gagcggtctg acagtggacg ccctgcacaa ggtgggccag atcgtgtttg 300

agttcgtggg cgagcctgag ctcatggacg tgcatgtctt ctgcacagac agcatccagg 360

ggacccccgt ggagagcgac gaaatgcgcc catgctggtt ccagctggat cagatcccct 420

tcaaggacat gtggcccgac gacagctact ggtttccact cctgcttcag aagaagaaat 480

tccacgggta cttcaagttc cagggtcagg acaccatcct ggactacaca ctccgcgagg 540

tggacacggt ctagcgggag cccagggcag cccctgggca ggagacgtgg ctgctgaaca 600

gccgcaaacc atcttcacct gggggcattg agtggcgcag agccgggttt catctggaat 660

taactggatg gaagggaaaa taaagctatc tagcggtgaa aaaaaaaaaa aaaaaaaaaa 720

aaaaaaaa 728

<210> 13
<211> 179
<212> PRT
<213> Homo sapiens

<400> 13

Met Ser Gly Ile Ser Pro Gln Gln Met Gly Glu Pro Glu Gly Ser Trp
1               5                   10                  15

Ser Gly Lys Asn Pro Gly Thr Met Gly Ala Ser Arg Leu Tyr Thr Leu
            20                  25                  30

Val Leu Val Leu Gln Pro Gln Arg Val Leu Leu Gly Met Lys Lys Arg
35 40 45

Gly Phe Gly Ala Gly Arg Trp Asn Gly Phe Gly Gly Lys Val Gln Glu
50 55 60

Gly Glu Thr Ile Glu Asp Gly Ala Arg Arg Glu Leu Gln Glu Glu Ser
65 70 75 80

Gly Leu Thr Val Asp Ala Leu His Lys Val Gly Gln Ile Val Phe Glu
85 90 95

Phe Val Gly Glu Pro Glu Leu Met Asp Val His Val Phe Cys Thr Asp
100 105 110

Ser Ile Gln Gly Thr Pro Val Glu Ser Asp Glu Met Arg Pro Cys Trp
115 120 125

Phe Gln Leu Asp Gln Ile Pro Phe Lys Asp Met Trp Pro Asp Asp Ser
130 135 140

Tyr Trp Phe Pro Leu Leu Leu Gln Lys Lys Lys Phe His Gly Tyr Phe
145 150 155 160

Lys Phe Gln Gly Gln Asp Thr Ile Leu Asp Tyr Thr Leu Arg Glu Val
165 170 175

Asp Thr Val

<210> 14
<211> 156
<212> PRT
<213> Homo sapiens

<400> 14

Met Gly Ala Ser Arg Leu Tyr Thr Leu Val Leu Val Leu Gln Pro Gln
1 5 10 15

Arg Val Leu Leu Gly Met Lys Lys Arg Gly Phe Gly Ala Gly Arg Trp
20 25 30

Asn Gly Phe Gly Gly Lys Val Gln Glu Gly Glu Thr Ile Glu Asp Gly
35 40 45

Ala Arg Arg Glu Leu Gln Glu Glu Ser Gly Leu Thr Val Asp Ala Leu
50 55 60

His Lys Val Gly Gln Ile Val Phe Glu Phe Val Gly Glu Pro Glu Leu
65 70 75 80

Met Asp Val His Val Phe Cys Thr Asp Ser Ile Gln Gly Thr Pro Val
85 90 95

Glu Ser Asp Glu Met Arg Pro Cys Trp Phe Gln Leu Asp Gln Ile Pro
100 105 110

Phe Lys Asp Met Trp Pro Asp Asp Ser Tyr Trp Phe Pro Leu Leu Leu
115 120 125

Gln Lys Lys Lys Phe His Gly Tyr Phe Lys Phe Gln Gly Gln Asp Thr
130 135 140

Ile Leu Asp Tyr Thr Leu Arg Glu Val Asp Thr Val
145 150 155

<210> 15
<211> 5156
<212> DNA
<213> Homo sapiens

<400> 15
agccaatggg agttcaggag gcggagcgcc tgtgggagcc ctggagggaa ctttcccagt 60

ccccgaggcg gatcgggtgt tgcatccatg gagcgagctg agagctcgag tacagaacct 120

gctaaggcca tcaaacctat tgatcggaag tcagtccatc agatttgctc tgggcaggtg 180

gtactgagtc taagcactgc ggtaaaggag ttagtagaaa acagtctgga tgctggtgcc 240

actaatattg atctaaagct taaggactat ggagtggatc ttattgaagt ttcagacaat 300

ggatgtgggg tagaagaaga aaacttcgaa ggcttaactc tgaaacatca cacatctaag 360

attcaagagt ttgccgacct aactcaggtt gaaacttttg gctttcgggg ggaagctctg 420

agctcacttt gtgcactgag cgatgtcacc atttctacct gccacgcatc ggcgaaggtt 480

ggaactcgac tgatgtttga tcacaatggg aaaattatcc agaaaacccc ctacccccgc 540

cccagaggga ccacagtcag cgtgcagcag ttattttcca cactacctgt gcgccataag 600

gaatttcaaa ggaatattaa gaaggagtat gccaaaatgg tccaggtctt acatgcatac 660

tgtatcattt cagcaggcat ccgtgtaagt tgcaccaatc agcttggaca aggaaaacga 720

cagcctgtgg tatgcacagg tggaagcccc agcataaagg aaaatatcgg ctctgtgttt 780

gggcagaagc agttgcaaag cctcattcct tttgttcagc tgccccctag tgactccgtg 840

tgtgaagagt acggtttgag ctgttccgat gctctgcata atcttttta catctcaggt 900

ttcatttcac aatgcacgca tggagttgga aggagttcaa cagacagaca gtttttcttt 960

atcaaccggc ggccttgtga cccagcaaag gtctgcagac tcgtgaatga ggtctaccac 1020

atgtataatc gacaccagta tccatttgtt gttcttaaca tttctgttga ttcagaatgc 1080

gttgatatca atgttactcc agataaaagg caaattttgc tacaagagga aaagcttttg 1140

ttggcagttt taaagacctc tttgatagga atgtttgata gtgatgtcaa caagctaaat 1200

gtcagtcagc agccactgct ggatgttgaa ggtaacttaa taaaaatgca tgcagcggat 1260

ttggaaaagc ccatggtaga aaagcaggat caatcccctt cattaaggac tggagaagaa 1320

aaaaaagacg tgtccatttc cagactgcga gaggcctttt ctcttcgtca cacaacagag 1380

aacaagcctc acagcccaaa gactccagaa ccaagaagga gccctctagg acagaaaagg 1440

ggtatgctgt cttctagcac ttcaggtgcc atctctgaca aaggcgtcct gagacctcag 1500

aaagaggcag tgagttccag tcacggaccc agtgacccta cggacagagc ggaggtggag 1560

aaggactcgg ggcacggcag cacttccgtg gattctgagg ggttcagcat cccagacacg 1620

ggcagtcact gcagcagcga gtatgcggcc agctccccag ggacagggg ctcgcaggaa 1680

catgtggact ctcaggagaa agcgcctaaa actgacgact ctttttcaga tgtggactgc 1740

cattcaaacc aggaagatac cggatgtaaa tttcgagttt tgcctcagcc aactaatctc 1800

gcaaccccaa acacaaagcg ttttaaaaaa gaagaaattc tttccagttc tgacatttgt 1860

caaaagttag taaatactca ggacatgtca gcctctcagg ttgatgtagc tgtgaaaatt 1920

aataagaaag ttgtgcccct ggacttttct atgagttctt tagctaaacg aataaagcag 1980

ttacatcatg aagcacagca aagtgaaggg gaacagaatt acaggaagtt tagggcaaag 2040

atttgtcctg gagaaaatca agcagccgaa gatgaactaa gaaaagagat aagtaaaacg 2100

atgtttgcag aaatggaaat cattggtcag tttaacctgg gatttataat aaccaaactg 2160

aatgaggata tcttcatagt ggaccagcat gccacggacg agaagtataa cttcgagatg 2220

ctgcagcagc acaccgtgct ccaggggcag aggctcatag cacctcagac tctcaactta 2280

actgctgtta atgaagctgt tctgatagaa aatctggaaa tatttagaaa gaatggcttt 2340

gattttgtta tcgatgaaaa tgctccagtc actgaaaggg ctaaactgat ttccttgcca 2400

actagtaaaa actggacctt cggaccccag gacgtcgatg aactgatctt catgctgagc 2460

gacagccctg gggtcatgtg ccggccttcc cgagtcaagc agatgtttgc ctccagagcc 2520

tgccggaagt cggtgatgat tgggactgct cttaacacaa gcgagatgaa gaaactgatc 2580

acccacatgg gggagatgga ccacccctgg aactgtcccc atggaaggcc aaccatgaga 2640

cacatcgcca acctgggtgt catttctcag aactgaccgt agtcactgta tggaataatt 2700

ggttttatcg cagattttta tgttttgaaa gacagagtct tcactaacct tttttgtttt 2760

aaaatgaacc tgctacttaa aaaaaataca catcacaccc atttaaaagt gatcttgaga 2820

accttttcaa accagatgga gcattgcttg caaatttttt ttctctatgt ttgcatgcgc 2880

tcgtgtgtgt gtgtccaggc aagaacacat tttataaaaa taagaacact tgggctgggc 2940
atggtggctc atgcctgtga tcgcagcact ttgggaggcc gaggccggcg gatcacctga 3000
gatcagaagt tcgagaccag cctgaccaac atggagaaac cctgcctcta ctaaaaatac 3060
aaaattagcc aggtgtgctg gcgcatgcct gtaatccccg ctacccagga ggctgaggca 3120
ggagaatcgc ttgaacccgg gagacggagg ttgcagtgaa ccgagattgc gccactgcgc 3180
tccagcctgg gtgagataga acaagactgt gtctcaaaaa acaaaacaaa acaaaacaaa 3240
aaaaaaaaaa ccaaaccact ttggaagtta ctcaggcctc tgctctggct ggacatagtt 3300
tagtctataa ctttcaaccc ttaatgataa ttaaattcat ctttgtttaa tttcataaat 3360
ttaaaagtag ggtccttttc agttagtgat tctcagccct gattcacatt aaatttttaa 3420
acacggggga ttctctgccc ggctggaaga aaatgactgg atgggacagg ggtcactatt 3480
tgaaacattc ctctgtgcgg ccaaggtcgc aaaatgctgt cctcgcaggg gaacaaaaag 3540
agtttgattt cccataattt gatgctgtga tttggtttcc tcaggatgtg aactgtagaa 3600
cattccagtt actggccttg aatggttctg ggaatataag aatccctgtc tgtcttttca 3660
aatagttttc atggaacctt gtcctgtttg aacttggctg aaaatggaag taaagatgcc 3720
ctcttggggg cccagagatg acagatgtgg ctccccctgc tgcccccacc ccttctccag 3780
actgtgggcg gctccccttc ctgctttaga atccctcaga tggaggaggc agtacagtag 3840
tcactgtgcc atcgtgtctg gcactgtgct ggcgtggtct gcaggatccc acttatgaac 3900
tctccagatt gggagctgtg gcaggataac agcccccaag acagctgtgt cctaatcccc 3960
agaacctgtg accacgctgc ctcacgtggc agaagggact cggcaggtgt gattgagtga 4020
aggatctttt tttttttttt ctttgagatg aagtttcgct cttgttgccc aggctggagt 4080
tcaatagcat gatctcagct cactgcagcc tctgcctccc aggttcaagt gattctccca 4140
cctcagcctc ccgagtagct gggattacag gtgtccagaa ccatactggc taatttttgt 4200
atttttagta gagacagggt ttcaccatgt tgaccaggct ggtctcgaac tcctgacctc 4260
aggtgatccg accgcctcgg cctcccaaag tgctgggatt acaggtgtga gccatcatgc 4320
ctggctgagt taaggatctt gcaacagaga gattatcctg gattgtctgg gtgggcccag 4380
tccattgggt gagtccttca aaggtggaga cctttccctg ctggccagag agaggctgtc 4440
ttgctggttt tggagatgga aggaggtacc actagtcaag gattgcaagc agtctctaga 4500
acagggattc caacactccg gacacagacc agtagtggtc catggcctat taggaagtgg 4560
ggtgcacagc aggttagggg ccggcaagcc agcgaagctt catctgtatt tatagccact 4620
ccccgtcgct ggcgttacca cccgagctcc gcctcctgtc acatcagcgg tgggcattag 4680
attctcatag cagcacgagc cctattgtga actgcacaca cgagggatgt aggttgcacg 4740
ctccttatga gaatctgatg cctgatgatc tgtcactgtc tcccgtcacc cccagatggg 4800

gctgtctagt tgcaggaaaa caagctcagg gctcccactg agtctctgtg atggtgagtt 4860

gtagaattat ttaattatat gttacaatgt aataatagta gaaataaagt gcacaataaa 4920

tgcaatgcac ttgaatcgtc ctgaaaccat ccctccccga ccccaatcca tggaaaaatt 4980

gtgttccgcg aaaccggtct ctggtgccaa aaaggttggg gaccgcttct ggaaaagctg 5040

gaaaaggcaa gaaaacgcat tctctccctc agcctctgga aggaaccagc actgtgggac 5100

taatttacat actgtagggt aataaatttg tgttgcttcg aaccactaaa aaaaaa 5156

<210> 16
<211> 5283
<212> DNA
<213> Homo sapiens

<400> 16
agtccccgag gcggatcggg tgttgcatcc atggagcgag ctgagagctc gaggtgagcg 60

gggctcgcag tcttccggtg tcccctctcg cgcgccctct ttgagaccca cggcattcca 120

acctccctgg aaatgggggg aacatggccg aggcgcgtgg cgaggcagcc ccctggaggc 180

cccggagtgg cgacctcagt gttgcagcga tctcgtgccc cttgggtgcg ccttgaggcc 240

gagtacagaa cctgctaagg ccatcaaacc tattgatcgg aagtcagtcc atcagatttg 300

ctctgggcag gtggtactga gtctaagcac tgcggtaaag gagttagtag aaaacagtct 360

ggatgctggt gccactaata ttgatctaaa gcttaaggac tatggagtgg atcttattga 420

agtttcagac aatggatgtg ggtagaaga agaaaacttc gaaggcttaa ctctgaaaca 480

tcacacatct aagattcaag agtttgccga cctaactcag gttgaaactt ttggctttcg 540

gggggaagct ctgagctcac tttgtgcact gagcgatgtc accatttcta cctgccacgc 600

atcggcgaag gttggaactc gactgatgtt tgatcacaat gggaaaatta tccagaaaac 660

cccctacccc cgccccagag ggaccacagt cagcgtgcag cagttatttt ccacactacc 720

tgtgcgccat aaggaatttc aaaggaatat taagaaggag tatgccaaaa tggtccaggt 780

cttacatgca tactgtatca tttcagcagg catccgtgta agttgcacca atcagcttgg 840

acaaggaaaa cgacagcctg tggtatgcac aggtggaagc cccagcataa aggaaaatat 900

cggctctgtg tttgggcaga agcagttgca aagcctcatt ccttttgttc agctgccccc 960

tagtgactcc gtgtgtgaag agtacggttt gagctgttcc gatgctctgc ataatctttt 1020

ttacatctca ggtttcattt cacaatgcac gcatggagtt ggaaggagtt caacagacag 1080

acagtttttc tttatcaacc ggcggccttg tgacccagca aaggtctgca gactcgtgaa 1140

tgaggtctac cacatgtata atcgacacca gtatccattt gttgttctta acatttctgt 1200

tgattcagaa tgcgttgata tcaatgttac tccagataaa aggcaaattt tgctacaaga 1260

ggaaaagctt ttgttggcag ttttaaagac ctctttgata ggaatgtttg atagtgatgt 1320

caacaagcta aatgtcagtc agcagccact gctggatgtt gaaggtaact taataaaaat 1380
gcatgcagcg gatttggaaa agcccatggt agaaaagcag gatcaatccc cttcattaag 1440
gactggagaa gaaaaaaaag acgtgtccat ttccagactg cgagaggcct tttctcttcg 1500
tcacacaaca gagaacaagc ctcacagccc aaagactcca gaaccaagaa ggagccctct 1560
aggacagaaa aggggtatgc tgtcttctag cacttcaggt gccatctctg acaaaggcgt 1620
cctgagacct cagaaagagg cagtgagttc cagtcacgga cccagtgacc ctacggacag 1680
agcggaggtg gagaaggact cggggcacgg cagcacttcc gtggattctg aggggttcag 1740
catcccagac acgggcagtc actgcagcag cgagtatgcg gccagctccc caggggacag 1800
gggctcgcag gaacatgtgg actctcagga gaaagcgcct aaaactgacg actctttttc 1860
agatgtggac tgccattcaa accaggaaga taccggatgt aaatttcgag ttttgcctca 1920
gccaactaat ctcgcaaccc caaacacaaa gcgttttaaa aaagaagaaa ttctttccag 1980
ttctgacatt tgtcaaaagt tagtaaatac tcaggacatg tcagcctctc aggttgatgt 2040
agctgtgaaa attaataaga aagttgtgcc cctggacttt tctatgagtt ctttagctaa 2100
acgaataaag cagttacatc atgaagcaca gcaaagtgaa ggggaacaga attacaggaa 2160
gtttagggca aagatttgtc ctggagaaaa tcaagcagcc gaagatgaac taagaaaaga 2220
gataagtaaa acgatgtttg cagaaatgga aatcattggt cagtttaacc tgggatttat 2280
aataaccaaa ctgaatgagg atatcttcat agtggaccag catgccacgg acgagaagta 2340
taacttcgag atgctgcagc agcacaccgt gctccagggg cagaggctca tagcacctca 2400
gactctcaac ttaactgctg ttaatgaagc tgttctgata gaaaatctgg aaatatttag 2460
aaagaatggc tttgattttg ttatcgatga aaatgctcca gtcactgaaa gggctaaact 2520
gatttccttg ccaactagta aaaactggac cttcggaccc caggacgtcg atgaactgat 2580
cttcatgctg agcgacagcc ctggggtcat gtgccggcct tcccgagtca agcagatgtt 2640
tgcctccaga gcctgccgga agtcggtgat gattgggact gctcttaaca caagcgagat 2700
gaagaaactg atcacccaca tgggggagat ggaccacccc tggaactgtc cccatggaag 2760
gccaaccatg agacacatcg ccaacctggg tgtcatttct cagaactgac cgtagtcact 2820
gtatggaata attggtttta tcgcagattt ttatgttttg aaagacagag tcttcactaa 2880
ccttttttgt tttaaaatga acctgctact taaaaaaaat acacatcaca cccatttaaa 2940
agtgatcttg agaacctttt caaaccagat ggagcattgc ttgcaaattt tttttctcta 3000
tgtttgcatg cgctcgtgtg tgtgtgtcca ggcaagaaca cattttataa aaataagaac 3060
acttgggctg ggcatggtgg ctcatgcctg tgatcgcagc actttgggag gccgaggccg 3120
gcggatcacc tgagatcaga agttcgagac cagcctgacc aacatggaga aaccctgcct 3180

ctactaaaaa tacaaaatta gccaggtgtg ctggcgcatg cctgtaatcc ccgctaccca 3240
ggaggctgag gcaggagaat cgcttgaacc cgggagacgg aggttgcagt gaaccgagat 3300
tgcgccactg cgctccagcc tgggtgagat agaacaagac tgtgtctcaa aaaacaaaac 3360
aaaacaaaac aaaaaaaaaa aaaccaaacc actttggaag ttactcaggc ctctgctctg 3420
gctggacata gtttagtcta taactttcaa cccttaatga taattaaatt catctttgtt 3480
taatttcata aatttaaaag tagggtcctt ttcagttagt gattctcagc cctgattcac 3540
attaaatttt taaacacggg ggattctctg cccggctgga agaaaatgac tggatgggac 3600
aggggtcact atttgaaaca ttcctctgtg cggccaaggt cgcaaaatgc tgtcctcgca 3660
ggggaacaaa aagagtttga tttcccataa tttgatgctg tgatttggtt tcctcaggat 3720
gtgaactgta gaacattcca gttactggcc ttgaatggtt ctgggaatat aagaatccct 3780
gtctgtcttt tcaaatagtt ttcatggaac cttgtcctgt ttgaacttgg ctgaaaatgg 3840
aagtaaagat gccctcttgg gggcccagag atgacagatg tggctccccc tgctgccccc 3900
accccttctc cagactgtgg gcggctcccc ttcctgcttt agaatccctc agatggagga 3960
ggcagtacag tagtcactgt gccatcgtgt ctggcactgt gctggcgtgg tctgcaggat 4020
cccacttatg aactctccag attgggagct gtggcaggat aacagccccc aagacagctg 4080
tgtcctaatc cccagaacct gtgaccacgc tgcctcacgt ggcagaaggg actcggcagg 4140
tgtgattgag tgaaggatct tttttttttt tttctttgag atgaagtttc gctcttgttg 4200
cccaggctgg agttcaatag catgatctca gctcactgca gcctctgcct cccaggttca 4260
agtgattctc ccacctcagc ctcccgagta gctgggatta caggtgtcca gaaccatact 4320
ggctaatttt tgtattttta gtagagacag ggtttcacca tgttgaccag gctggtctcg 4380
aactcctgac ctcaggtgat ccgaccgcct cggcctccca aagtgctggg attacaggtg 4440
tgagccatca tgcctggctg agttaaggat cttgcaacag agagattatc ctggattgtc 4500
tgggtgggcc cagtccattg ggtgagtcct tcaaaggtgg agacctttcc ctgctggcca 4560
gagagaggct gtcttgctgg ttttggagat ggaaggaggt accactagtc aaggattgca 4620
agcagtctct agaacaggga ttccaacact ccggacacag accagtagtg gtccatggcc 4680
tattaggaag tggggtgcac agcaggttag gggccggcaa gccagcgaag cttcatctgt 4740
atttatagcc actccccgtc gctggcgtta ccacccgagc tccgcctcct gtcacatcag 4800
cggtgggcat tagattctca tagcagcacg agccctattg tgaactgcac acacgaggga 4860
tgtaggttgc acgctcctta tgagaatctg atgcctgatg atctgtcact gtctcccgtc 4920
acccccagat ggggctgtct agttgcagga aaacaagctc agggctccca ctgagtctct 4980
gtgatggtga gttgtagaat tatttaatta tatgttacaa tgtaataata gtagaaataa 5040
agtgcacaat aaatgcaatg cacttgaatc gtcctgaaac catccctccc cgaccccaat 5100

ccatggaaaa attgtgttcc gcgaaaccgg tctctggtgc caaaaaggtt ggggaccgct 5160

tctggaaaag ctggaaaagg caagaaaacg cattctctcc ctcagcctct ggaaggaacc 5220

agcactgtgg gactaattta catactgtag ggtaataaat ttgtgttgct tcgaaccact 5280

aaa 5283

<210> 17
<211> 4913
<212> DNA
<213> Homo sapiens

<400> 17
agccaatggg agttcaggag gcggagcgcc tgtgggagcc ctggagggaa ctttcccagt 60

ccccgaggcg gatcgggtgt tgcatccatg gagcgagctg agagctcgag atctaaagct 120

taaggactat ggagtggatc ttattgaagt ttcagacaat ggatgtgggg tagaagaaga 180

aaacttcgaa ggcttaacga tgtcaccatt tctacctgcc acgcatcggc gaaggttgga 240

actcgactga tgtttgatca caatgggaaa attatccaga aaacccccta cccccgcccc 300

agagggacca cagtcagcgt gcagcagtta ttttccacac tacctgtgcg ccataaggaa 360

tttcaaagga atattaagaa ggagtatgcc aaaatggtcc aggtcttaca tgcatactgt 420

atcatttcag caggcatccg tgtaagttgc accaatcagc ttggacaagg aaaacgacag 480

cctgtggtat gcacaggtgg aagccccagc ataaaggaaa atatcggctc tgtgtttggg 540

cagaagcagt tgcaaagcct cattcctttt gttcagctgc cccctagtga ctccgtgtgt 600

gaagagtacg gtttgagctg ttccgatgct ctgcataatc tttttacat ctcaggtttc 660

atttcacaat gcacgcatgg agttggaagg agttcaacag acagacagtt tttctttatc 720

aaccggcggc cttgtgaccc agcaaaggtc tgcagactcg tgaatgaggt ctaccacatg 780

tataatcgac accagtatcc atttgttgtt cttaacattt ctgttgattc agaatgcgtt 840

gatatcaatg ttactccaga taaaaggcaa attttgctac aagaggaaaa gcttttgttg 900

gcagttttaa agacctcttt gataggaatg tttgatagtg atgtcaacaa gctaaatgtc 960

agtcagcagc cactgctgga tgttgaaggt aacttaataa aaatgcatgc agcggatttg 1020

gaaaagccca tggtagaaaa gcaggatcaa tccccttcat taaggactgg agaagaaaaa 1080

aaagacgtgt ccatttccag actgcgagag gccttttctc ttcgtcacac aacagagaac 1140

aagcctcaca gcccaaagac tccagaacca agaaggagcc ctctaggaca gaaaaggggt 1200

atgctgtctt ctagcacttc aggtgccatc tctgacaaag gcgtcctgag acctcagaaa 1260

gaggcagtga gttccagtca cggacccagt gaccctacgg acagagcgga ggtggagaag 1320

gactcggggc acggcagcac ttccgtggat tctgaggggt tcagcatccc agacacgggc 1380

agtcactgca gcagcgagta tgcggccagc tccccagggg acaggggctc gcaggaacat 1440

gtggactctc aggagaaagc gcctaaaact gacgactctt tttcagatgt ggactgccat 1500
tcaaaccagg aagataccgg atgtaaattt cgagttttgc ctcagccaac taatctcgca 1560
accccaaaca caaagcgttt taaaaaagaa gaaattcttt ccagttctga catttgtcaa 1620
aagttagtaa atactcagga catgtcagcc tctcaggttg atgtagctgt gaaaattaat 1680
aagaaagttg tgcccctgga cttttctatg agttctttag ctaaacgaat aaagcagtta 1740
catcatgaag cacagcaaag tgaaggggaa cagaattaca ggaagtttag ggcaaagatt 1800
tgtcctggag aaaatcaagc agccgaagat gaactaagaa aagagataag taaaacgatg 1860
tttgcagaaa tggaaatcat tggtcagttt aacctgggat ttataataac caaactgaat 1920
gaggatatct tcatagtgga ccagcatgcc acggacgaga agtataactt cgagatgctg 1980
cagcagcaca ccgtgctcca ggggcagagg ctcatagcac ctcagactct caacttaact 2040
gctgttaatg aagctgttct gatagaaaat ctggaaatat ttagaaagaa tggctttgat 2100
tttgttatcg atgaaaatgc tccagtcact gaaagggcta aactgatttc cttgccaact 2160
agtaaaaact ggaccttcgg accccaggac gtcgatgaac tgatcttcat gctgagcgac 2220
agccctgggg tcatgtgccg gccttcccga gtcaagcaga tgtttgcctc cagagcctgc 2280
cggaagtcgg tgatgattgg gactgctctt aacacaagcg agatgaagaa actgatcacc 2340
cacatggggg agatggacca cccctggaac tgtccccatg gaaggccaac catgagacac 2400
atcgccaacc tgggtgtcat ttctcagaac tgaccgtagt cactgtatgg aataattggt 2460
tttatcgcag atttttatgt tttgaaagac agagtcttca ctaacctttt ttgttttaaa 2520
atgaacctgc tacttaaaaa aaatacacat cacacccatt taaaagtgat cttgagaacc 2580
ttttcaaacc agatggagca ttgcttgcaa attttttttc tctatgtttg catgcgctcg 2640
tgtgtgtgtg tccaggcaag aacacatttt ataaaaataa gaacacttgg gctgggcatg 2700
gtggctcatg cctgtgatcg cagcactttg ggaggccgag gccggcggat cacctgagat 2760
cagaagttcg agaccagcct gaccaacatg gagaaaccct gcctctacta aaaatacaaa 2820
attagccagg tgtgctggcg catgcctgta atccccgcta cccaggaggc tgaggcagga 2880
gaatcgcttg aacccgggag acggaggttg cagtgaaccg agattgcgcc actgcgctcc 2940
agcctgggtg agatagaaca agactgtgtc tcaaaaaaca aaacaaaaca aaacaaaaaa 3000
aaaaaaacca aaccactttg gaagttactc aggcctctgc tctggctgga catagtttag 3060
tctataactt tcaaccctta atgataatta aattcatctt tgtttaattt cataaattta 3120
aaagtagggt ccttttcagt tagtgattct cagccctgat tcacattaaa tttttaaaca 3180
cgggggattc tctgcccggc tggaagaaaa tgactggatg ggacaggggt cactatttga 3240
aacattcctc tgtgcggcca aggtcgcaaa atgctgtcct cgcaggggaa caaaaagagt 3300

ttgatttccc ataatttgat gctgtgattt ggtttcctca ggatgtgaac tgtagaacat 3360

tccagttact ggccttgaat ggttctggga atataagaat ccctgtctgt cttttcaaat 3420

agttttcatg gaaccttgtc ctgtttgaac ttggctgaaa atggaagtaa agatgccctc 3480

ttgggggccc agagatgaca gatgtggctc cccctgctgc ccccacccct tctccagact 3540

gtgggcggct cccccttcctg ctttagaatc cctcagatgg aggaggcagt acagtagtca 3600

ctgtgccatc gtgtctggca ctgtgctggc gtggtctgca ggatcccact tatgaactct 3660

ccagattggg agctgtggca ggataacagc cccaagaca gctgtgtcct aatccccaga 3720

acctgtgacc acgctgcctc acgtggcaga agggactcgg caggtgtgat tgagtgaagg 3780

atcttttttt tttttttctt tgagatgaag tttcgctctt gttgcccagg ctggagttca 3840

atagcatgat ctcagctcac tgcagcctct gcctcccagg ttcaagtgat tctcccacct 3900

cagcctcccg agtagctggg attacaggtg tccagaacca tactggctaa tttttgtatt 3960

tttagtagag acagggtttc accatgttga ccaggctggt ctcgaactcc tgacctcagg 4020

tgatccgacc gcctcggcct cccaaagtgc tgggattaca ggtgtgagcc atcatgcctg 4080

gctgagttaa ggatcttgca acagagagat tatcctggat tgtctgggtg ggcccagtcc 4140

attgggtgag tccttcaaag gtggagacct ttccctgctg gccagagaga ggctgtcttg 4200

ctggttttgg agatggaagg aggtaccact agtcaaggat tgcaagcagt ctctagaaca 4260

gggattccaa cactccggac acagaccagt agtggtccat ggcctattag gaagtggggt 4320

gcacagcagg ttaggggccg gcaagccagc gaagcttcat ctgtatttat agccactccc 4380

cgtcgctggc gttaccaccc gagctccgcc tcctgtcaca tcagcggtgg gcattagatt 4440

ctcatagcag cacgagccct attgtgaact gcacacacga gggatgtagg ttgcacgctc 4500

cttatgagaa tctgatgcct gatgatctgt cactgtctcc cgtcaccccc agatggggct 4560

gtctagttgc aggaaaacaa gctcagggct cccactgagt ctctgtgatg gtgagttgta 4620

gaattattta attatatgtt acaatgtaat aatagtagaa ataaagtgca caataaatgc 4680

aatgcacttg aatcgtcctg aaaccatccc tccccgaccc caatccatgg aaaaattgtg 4740

ttccgcgaaa ccggtctctg gtgccaaaaa ggttggggac cgcttctgga aaagctggaa 4800

aaggcaagaa aacgcattct ctccctcagc ctctggaagg aaccagcact gtgggactaa 4860

tttacatact gtagggtaat aaatttgtgt tgcttcgaac cactaaaaaa aaa 4913

<210> 18
<211> 862
<212> PRT
<213> Homo sapiens

<400> 18

Met Glu Arg Ala Glu Ser Ser Ser Thr Glu Pro Ala Lys Ala Ile Lys

```
1               5                   10                  15

Pro Ile Asp Arg Lys Ser Val His Gln Ile Cys Ser Gly Gln Val Val
            20                  25                  30

Leu Ser Leu Ser Thr Ala Val Lys Glu Leu Val Glu Asn Ser Leu Asp
        35                  40                  45

Ala Gly Ala Thr Asn Ile Asp Leu Lys Leu Lys Asp Tyr Gly Val Asp
    50                  55                  60

Leu Ile Glu Val Ser Asp Asn Gly Cys Gly Val Glu Glu Glu Asn Phe
65                  70                  75                  80

Glu Gly Leu Thr Leu Lys His His Thr Ser Lys Ile Gln Glu Phe Ala
                85                  90                  95

Asp Leu Thr Gln Val Glu Thr Phe Gly Phe Arg Gly Glu Ala Leu Ser
            100                 105                 110

Ser Leu Cys Ala Leu Ser Asp Val Thr Ile Ser Thr Cys His Ala Ser
        115                 120                 125

Ala Lys Val Gly Thr Arg Leu Met Phe Asp His Asn Gly Lys Ile Ile
    130                 135                 140

Gln Lys Thr Pro Tyr Pro Arg Pro Arg Gly Thr Thr Val Ser Val Gln
145                 150                 155                 160

Gln Leu Phe Ser Thr Leu Pro Val Arg His Lys Glu Phe Gln Arg Asn
                165                 170                 175

Ile Lys Lys Glu Tyr Ala Lys Met Val Gln Val Leu His Ala Tyr Cys
            180                 185                 190

Ile Ile Ser Ala Gly Ile Arg Val Ser Cys Thr Asn Gln Leu Gly Gln
        195                 200                 205

Gly Lys Arg Gln Pro Val Val Cys Thr Gly Gly Ser Pro Ser Ile Lys
    210                 215                 220

Glu Asn Ile Gly Ser Val Phe Gly Gln Lys Gln Leu Gln Ser Leu Ile
225                 230                 235                 240

Pro Phe Val Gln Leu Pro Pro Ser Asp Ser Val Cys Glu Glu Tyr Gly
                245                 250                 255
```

Leu Ser Cys Ser Asp Ala Leu His Asn Leu Phe Tyr Ile Ser Gly Phe
260 265 270

Ile Ser Gln Cys Thr His Gly Val Gly Arg Ser Ser Thr Asp Arg Gln
275 280 285

Phe Phe Phe Ile Asn Arg Arg Pro Cys Asp Pro Ala Lys Val Cys Arg
290 295 300

Leu Val Asn Glu Val Tyr His Met Tyr Asn Arg His Gln Tyr Pro Phe
305 310 315 320

Val Val Leu Asn Ile Ser Val Asp Ser Glu Cys Val Asp Ile Asn Val
325 330 335

Thr Pro Asp Lys Arg Gln Ile Leu Leu Gln Glu Glu Lys Leu Leu Leu
340 345 350

Ala Val Leu Lys Thr Ser Leu Ile Gly Met Phe Asp Ser Asp Val Asn
355 360 365

Lys Leu Asn Val Ser Gln Gln Pro Leu Leu Asp Val Glu Gly Asn Leu
370 375 380

Ile Lys Met His Ala Ala Asp Leu Glu Lys Pro Met Val Glu Lys Gln
385 390 395 400

Asp Gln Ser Pro Ser Leu Arg Thr Gly Glu Glu Lys Lys Asp Val Ser
405 410 415

Ile Ser Arg Leu Arg Glu Ala Phe Ser Leu Arg His Thr Thr Glu Asn
420 425 430

Lys Pro His Ser Pro Lys Thr Pro Glu Pro Arg Arg Ser Pro Leu Gly
435 440 445

Gln Lys Arg Gly Met Leu Ser Ser Ser Thr Ser Gly Ala Ile Ser Asp
450 455 460

Lys Gly Val Leu Arg Pro Gln Lys Glu Ala Val Ser Ser Ser His Gly
465 470 475 480

Pro Ser Asp Pro Thr Asp Arg Ala Glu Val Glu Lys Asp Ser Gly His
485 490 495

Gly Ser Thr Ser Val Asp Ser Glu Gly Phe Ser Ile Pro Asp Thr Gly
500 505 510

Ser His Cys Ser Ser Glu Tyr Ala Ala Ser Ser Pro Gly Asp Arg Gly
515 520 525

Ser Gln Glu His Val Asp Ser Gln Glu Lys Ala Pro Lys Thr Asp Asp
530 535 540

Ser Phe Ser Asp Val Asp Cys His Ser Asn Gln Glu Asp Thr Gly Cys
545 550 555 560

Lys Phe Arg Val Leu Pro Gln Pro Thr Asn Leu Ala Thr Pro Asn Thr
565 570 575

Lys Arg Phe Lys Lys Glu Glu Ile Leu Ser Ser Ser Asp Ile Cys Gln
580 585 590

Lys Leu Val Asn Thr Gln Asp Met Ser Ala Ser Gln Val Asp Val Ala
595 600 605

Val Lys Ile Asn Lys Lys Val Val Pro Leu Asp Phe Ser Met Ser Ser
610 615 620

Leu Ala Lys Arg Ile Lys Gln Leu His His Glu Ala Gln Gln Ser Glu
625 630 635 640

Gly Glu Gln Asn Tyr Arg Lys Phe Arg Ala Lys Ile Cys Pro Gly Glu
645 650 655

Asn Gln Ala Ala Glu Asp Glu Leu Arg Lys Glu Ile Ser Lys Thr Met
660 665 670

Phe Ala Glu Met Glu Ile Ile Gly Gln Phe Asn Leu Gly Phe Ile Ile
675 680 685

Thr Lys Leu Asn Glu Asp Ile Phe Ile Val Asp Gln His Ala Thr Asp
690 695 700

Glu Lys Tyr Asn Phe Glu Met Leu Gln Gln His Thr Val Leu Gln Gly
705 710 715 720

Gln Arg Leu Ile Ala Pro Gln Thr Leu Asn Leu Thr Ala Val Asn Glu
725 730 735

Ala Val Leu Ile Glu Asn Leu Glu Ile Phe Arg Lys Asn Gly Phe Asp
740 745 750

Phe Val Ile Asp Glu Asn Ala Pro Val Thr Glu Arg Ala Lys Leu Ile
755 760 765

Ser Leu Pro Thr Ser Lys Asn Trp Thr Phe Gly Pro Gln Asp Val Asp
770 775 780

Glu Leu Ile Phe Met Leu Ser Asp Ser Pro Gly Val Met Cys Arg Pro
785 790 795 800

Ser Arg Val Lys Gln Met Phe Ala Ser Arg Ala Cys Arg Lys Ser Val
805 810 815

Met Ile Gly Thr Ala Leu Asn Thr Ser Glu Met Lys Lys Leu Ile Thr
820 825 830

His Met Gly Glu Met Asp His Pro Trp Asn Cys Pro His Gly Arg Pro
835 840 845

Thr Met Arg His Ile Ala Asn Leu Gly Val Ile Ser Gln Asn
850 855 860

<210> 19
<211> 727
<212> PRT
<213> Homo sapiens

<400> 19

Met Phe Asp His Asn Gly Lys Ile Ile Gln Lys Thr Pro Tyr Pro Arg
1 5 10 15

Pro Arg Gly Thr Thr Val Ser Val Gln Gln Leu Phe Ser Thr Leu Pro
20 25 30

Val Arg His Lys Glu Phe Gln Arg Asn Ile Lys Lys Glu Tyr Ala Lys
35 40 45

Met Val Gln Val Leu His Ala Tyr Cys Ile Ile Ser Ala Gly Ile Arg
50 55 60

Val Ser Cys Thr Asn Gln Leu Gly Gln Gly Lys Arg Gln Pro Val Val
65 70 75 80

Cys Thr Gly Gly Ser Pro Ser Ile Lys Glu Asn Ile Gly Ser Val Phe
85 90 95

Gly Gln Lys Gln Leu Gln Ser Leu Ile Pro Phe Val Gln Leu Pro Pro
100 105 110

Ser Asp Ser Val Cys Glu Glu Tyr Gly Leu Ser Cys Ser Asp Ala Leu
115 120 125

```
His Asn Leu Phe Tyr Ile Ser Gly Phe Ile Ser Gln Cys Thr His Gly
    130                 135                 140

Val Gly Arg Ser Ser Thr Asp Arg Gln Phe Phe Phe Ile Asn Arg Arg
145                 150                 155                 160

Pro Cys Asp Pro Ala Lys Val Cys Arg Leu Val Asn Glu Val Tyr His
                165                 170                 175

Met Tyr Asn Arg His Gln Tyr Pro Phe Val Val Leu Asn Ile Ser Val
            180                 185                 190

Asp Ser Glu Cys Val Asp Ile Asn Val Thr Pro Asp Lys Arg Gln Ile
        195                 200                 205

Leu Leu Gln Glu Glu Lys Leu Leu Leu Ala Val Leu Lys Thr Ser Leu
    210                 215                 220

Ile Gly Met Phe Asp Ser Asp Val Asn Lys Leu Asn Val Ser Gln Gln
225                 230                 235                 240

Pro Leu Leu Asp Val Glu Gly Asn Leu Ile Lys Met His Ala Ala Asp
                245                 250                 255

Leu Glu Lys Pro Met Val Glu Lys Gln Asp Gln Ser Pro Ser Leu Arg
            260                 265                 270

Thr Gly Glu Glu Lys Lys Asp Val Ser Ile Ser Arg Leu Arg Glu Ala
        275                 280                 285

Phe Ser Leu Arg His Thr Thr Glu Asn Lys Pro His Ser Pro Lys Thr
    290                 295                 300

Pro Glu Pro Arg Arg Ser Pro Leu Gly Gln Lys Arg Gly Met Leu Ser
305                 310                 315                 320

Ser Ser Thr Ser Gly Ala Ile Ser Asp Lys Gly Val Leu Arg Pro Gln
                325                 330                 335

Lys Glu Ala Val Ser Ser Ser His Gly Pro Ser Asp Pro Thr Asp Arg
            340                 345                 350

Ala Glu Val Glu Lys Asp Ser Gly His Gly Ser Thr Ser Val Asp Ser
        355                 360                 365

Glu Gly Phe Ser Ile Pro Asp Thr Gly Ser His Cys Ser Ser Glu Tyr
```

370 375 380

Ala Ala Ser Ser Pro Gly Asp Arg Gly Ser Gln Glu His Val Asp Ser
385 390 395 400

Gln Glu Lys Ala Pro Lys Thr Asp Asp Ser Phe Ser Asp Val Asp Cys
405 410 415

His Ser Asn Gln Glu Asp Thr Gly Cys Lys Phe Arg Val Leu Pro Gln
420 425 430

Pro Thr Asn Leu Ala Thr Pro Asn Thr Lys Arg Phe Lys Lys Glu Glu
435 440 445

Ile Leu Ser Ser Ser Asp Ile Cys Gln Lys Leu Val Asn Thr Gln Asp
450 455 460

Met Ser Ala Ser Gln Val Asp Val Ala Val Lys Ile Asn Lys Lys Val
465 470 475 480

Val Pro Leu Asp Phe Ser Met Ser Ser Leu Ala Lys Arg Ile Lys Gln
485 490 495

Leu His His Glu Ala Gln Gln Ser Glu Gly Glu Gln Asn Tyr Arg Lys
500 505 510

Phe Arg Ala Lys Ile Cys Pro Gly Glu Asn Gln Ala Ala Glu Asp Glu
515 520 525

Leu Arg Lys Glu Ile Ser Lys Thr Met Phe Ala Glu Met Glu Ile Ile
530 535 540

Gly Gln Phe Asn Leu Gly Phe Ile Ile Thr Lys Leu Asn Glu Asp Ile
545 550 555 560

Phe Ile Val Asp Gln His Ala Thr Asp Glu Lys Tyr Asn Phe Glu Met
565 570 575

Leu Gln Gln His Thr Val Leu Gln Gly Gln Arg Leu Ile Ala Pro Gln
580 585 590

Thr Leu Asn Leu Thr Ala Val Asn Glu Ala Val Leu Ile Glu Asn Leu
595 600 605

Glu Ile Phe Arg Lys Asn Gly Phe Asp Phe Val Ile Asp Glu Asn Ala
610 615 620

Pro Val Thr Glu Arg Ala Lys Leu Ile Ser Leu Pro Thr Ser Lys Asn
625 630 635 640

Trp Thr Phe Gly Pro Gln Asp Val Asp Glu Leu Ile Phe Met Leu Ser
645 650 655

Asp Ser Pro Gly Val Met Cys Arg Pro Ser Arg Val Lys Gln Met Phe
660 665 670

Ala Ser Arg Ala Cys Arg Lys Ser Val Met Ile Gly Thr Ala Leu Asn
675 680 685

Thr Ser Glu Met Lys Lys Leu Ile Thr His Met Gly Glu Met Asp His
690 695 700

Pro Trp Asn Cys Pro His Gly Arg Pro Thr Met Arg His Ile Ala Asn
705 710 715 720

Leu Gly Val Ile Ser Gln Asn
725

<210> 20
<211> 756
<212> PRT
<213> Homo sapiens

<400> 20

Met Trp Gly Arg Arg Arg Lys Leu Arg Arg Leu Asn Asp Val Thr Ile
1 5 10 15

Ser Thr Cys His Ala Ser Ala Lys Val Gly Thr Arg Leu Met Phe Asp
20 25 30

His Asn Gly Lys Ile Ile Gln Lys Thr Pro Tyr Pro Arg Pro Arg Gly
35 40 45

Thr Thr Val Ser Val Gln Gln Leu Phe Ser Thr Leu Pro Val Arg His
50 55 60

Lys Glu Phe Gln Arg Asn Ile Lys Lys Glu Tyr Ala Lys Met Val Gln
65 70 75 80

Val Leu His Ala Tyr Cys Ile Ile Ser Ala Gly Ile Arg Val Ser Cys
85 90 95

Thr Asn Gln Leu Gly Gln Gly Lys Arg Gln Pro Val Val Cys Thr Gly
100 105 110

Gly Ser Pro Ser Ile Lys Glu Asn Ile Gly Ser Val Phe Gly Gln Lys
115 120 125

Gln Leu Gln Ser Leu Ile Pro Phe Val Gln Leu Pro Pro Ser Asp Ser
130 135 140

Val Cys Glu Glu Tyr Gly Leu Ser Cys Ser Asp Ala Leu His Asn Leu
145 150 155 160

Phe Tyr Ile Ser Gly Phe Ile Ser Gln Cys Thr His Gly Val Gly Arg
165 170 175

Ser Ser Thr Asp Arg Gln Phe Phe Phe Ile Asn Arg Arg Pro Cys Asp
180 185 190

Pro Ala Lys Val Cys Arg Leu Val Asn Glu Val Tyr His Met Tyr Asn
195 200 205

Arg His Gln Tyr Pro Phe Val Val Leu Asn Ile Ser Val Asp Ser Glu
210 215 220

Cys Val Asp Ile Asn Val Thr Pro Asp Lys Arg Gln Ile Leu Leu Gln
225 230 235 240

Glu Glu Lys Leu Leu Leu Ala Val Leu Lys Thr Ser Leu Ile Gly Met
245 250 255

Phe Asp Ser Asp Val Asn Lys Leu Asn Val Ser Gln Gln Pro Leu Leu
260 265 270

Asp Val Glu Gly Asn Leu Ile Lys Met His Ala Ala Asp Leu Glu Lys
275 280 285

Pro Met Val Glu Lys Gln Asp Gln Ser Pro Ser Leu Arg Thr Gly Glu
290 295 300

Glu Lys Lys Asp Val Ser Ile Ser Arg Leu Arg Glu Ala Phe Ser Leu
305 310 315 320

Arg His Thr Thr Glu Asn Lys Pro His Ser Pro Lys Thr Pro Glu Pro
325 330 335

Arg Arg Ser Pro Leu Gly Gln Lys Arg Gly Met Leu Ser Ser Ser Thr
340 345 350

Ser Gly Ala Ile Ser Asp Lys Gly Val Leu Arg Pro Gln Lys Glu Ala
355 360 365

Val Ser Ser Ser His Gly Pro Ser Asp Pro Thr Asp Arg Ala Glu Val
370 375 380

Glu Lys Asp Ser Gly His Gly Ser Thr Ser Val Asp Ser Glu Gly Phe
385 390 395 400

Ser Ile Pro Asp Thr Gly Ser His Cys Ser Ser Glu Tyr Ala Ala Ser
405 410 415

Ser Pro Gly Asp Arg Gly Ser Gln Glu His Val Asp Ser Gln Glu Lys
420 425 430

Ala Pro Lys Thr Asp Asp Ser Phe Ser Asp Val Asp Cys His Ser Asn
435 440 445

Gln Glu Asp Thr Gly Cys Lys Phe Arg Val Leu Pro Gln Pro Thr Asn
450 455 460

Leu Ala Thr Pro Asn Thr Lys Arg Phe Lys Lys Glu Glu Ile Leu Ser
465 470 475 480

Ser Ser Asp Ile Cys Gln Lys Leu Val Asn Thr Gln Asp Met Ser Ala
485 490 495

Ser Gln Val Asp Val Ala Val Lys Ile Asn Lys Lys Val Val Pro Leu
500 505 510

Asp Phe Ser Met Ser Ser Leu Ala Lys Arg Ile Lys Gln Leu His His
515 520 525

Glu Ala Gln Gln Ser Glu Gly Glu Gln Asn Tyr Arg Lys Phe Arg Ala
530 535 540

Lys Ile Cys Pro Gly Glu Asn Gln Ala Ala Glu Asp Glu Leu Arg Lys
545 550 555 560

Glu Ile Ser Lys Thr Met Phe Ala Glu Met Glu Ile Ile Gly Gln Phe
565 570 575

Asn Leu Gly Phe Ile Ile Thr Lys Leu Asn Glu Asp Ile Phe Ile Val
580 585 590

Asp Gln His Ala Thr Asp Glu Lys Tyr Asn Phe Glu Met Leu Gln Gln
595 600 605

His Thr Val Leu Gln Gly Gln Arg Leu Ile Ala Pro Gln Thr Leu Asn
610 615 620

Leu Thr Ala Val Asn Glu Ala Val Leu Ile Glu Asn Leu Glu Ile Phe
625                 630                 635                 640

Arg Lys Asn Gly Phe Asp Phe Val Ile Asp Glu Asn Ala Pro Val Thr
                645                 650                 655

Glu Arg Ala Lys Leu Ile Ser Leu Pro Thr Ser Lys Asn Trp Thr Phe
            660                 665                 670

Gly Pro Gln Asp Val Asp Glu Leu Ile Phe Met Leu Ser Asp Ser Pro
        675                 680                 685

Gly Val Met Cys Arg Pro Ser Arg Val Lys Gln Met Phe Ala Ser Arg
    690                 695                 700

Ala Cys Arg Lys Ser Val Met Ile Gly Thr Ala Leu Asn Thr Ser Glu
705                 710                 715                 720

Met Lys Lys Leu Ile Thr His Met Gly Glu Met Asp His Pro Trp Asn
                725                 730                 735

Cys Pro His Gly Arg Pro Thr Met Arg His Ile Ala Asn Leu Gly Val
            740                 745                 750

Ile Ser Gln Asn
        755

<210> 21
<211> 1821
<212> DNA
<213> Homo sapiens

<400> 21
gagttgcggc gatgggcggg gcaggcgcgc ggggattggc gggatgcggc gcgccgcgcg 60

ggtgagacat cggtatccag gcacgataaa tttccaagtg gacacaatgt ctggtgtcaa 120

ctacagctgt tctccttctt ttcccagtat cctttgggtg cagtgagaca ccaggagagc 180

tgctgctttg ggggatggac aggggcagca ggaatgcctt tgtgttttcg cagtgaacct 240

ccttggcctg ggcgaagctg tgtggaccaa gcaagtcagg agtgtggcca tgttttctga 300

gcaggctgcc cagagggccc acactctact gtccccacca tcagccaaca atgccacctt 360

tgcccgggtg ccagtggcaa cctacaccaa ctcctcacaa cccttccggc taggagagcg 420

cagctttagc cggcagtatg cccacattta tgccacccgc ctcatccaaa tgagaccctt 480

cctggagaac cgggcccagc agcactgggg cagtggagtg ggagtgaaga agctgtgtga 540

actgcagcct gaggagaagt gctgtgtggt gggcactctg ttcaaggcca tgccgctgca 600

gccctccatc ctgcgggagg tcagcgagga gcacaacctg ctcccccagc ctcctcggag 660

taaatacata cacccagatg acgagctggt cttggaagat gaactgcagc gtatcaaact 720

aaaaggcacc attgacgtgt caaagctggt tacggggact gtcctggctg tgtttggctc 780

cgtgagagac gacgggaagt ttctggtgga ggactattgc tttgctgacc ttgctcccca 840

gaagcccgca cccccacttg acacagatag gtttgtgcta ctggtgtccg gcctgggcct 900

gggtggcggt ggaggcgaga gcctgctggg cacccagctg ctggtggatg tggtgacggg 960

gcagcttggg gacgaagggg agcagtgcag cgccgcccac gtctcccggg ttatcctcgc 1020

tggcaacctc ctcagccaca gcacccagag cagggattct atcaataagg ccaaatacct 1080

caccaagaaa acccaggcag ccagcgtgga ggctgttaag atgctggatg agatcctcct 1140

gcagctgagc gcctcagtgc ccgtggacgt gatgccaggc gagtttgatc ccaccaatta 1200

cacgctcccc cagcagcccc tccacccctg catgttcccg ctggccactg cctactccac 1260

gctccagctg gtcaccaacc cctaccaggc caccattgat ggagtcagat tttggggac 1320

atcaggacag aacgtgagtg acattttccg atacagcagc atggaggatc acttggagat 1380

cctggagtgg accctgcggg tccgtcacat cagccccaca gcccctgaca ctctaggttg 1440

ttacccttc tacaaaactg acccgttcat cttcccagag tgcccgcatg tctacttttg 1500

tggcaacacc cccagctttg gctccaaaat catccgaggt cctgaggacc agacagtgct 1560

gttggtgact gtccctgact tcagtgccac gcagaccgcc tgccttgtga acctgcgcag 1620

cctggcctgc cagcccatca gcttctcggg cttcggggca gaggacgatg acctgggagg 1680

cctggggctg ggcccctgac tcaaaaaagt ggttttgacc agagaggccc agatggaggc 1740

tgttcattcc ctgcagtgtc ggcattgtaa ataaagcctg agcacttgct gatgcgagcc 1800

ttgaaaaaaa aaaaaaaaaa a 1821

<210> 22
<211> 1648
<212> DNA
<213> Homo sapiens

<400> 22
gagttgcggc gatgggcggg gcaggcgcgc ggggattggc gggatgcggc gcgccgcgcg 60

tgaacctcct tggcctgggc gaagctgtgt ggaccaagca agtcaggagt gtggccatgt 120

tttctgagca ggctgcccag agggcccaca ctctactgtc cccaccatca gccaacaatg 180

ccacctttgc ccgggtgcca gtggcaacct acaccaactc ctcacaaccc ttccggctag 240

gagagcgcag ctttagccgg cagtatgccc acatttatgc cacccgcctc atccaaatga 300

gaccctteet ggagaaccgg gcccagcagc actggggcag tggagtggga gtgaagaagc 360

tgtgtgaact gcagcctgag gagaagtgct gtgtggtggg cactctgttc aaggccatgc 420

```
cgctgcagcc ctccatcctg cgggaggtca gcgaggagca caacctgctc ccccagcctc      480
ctcggagtaa atacatacac ccagatgacg agctggtctt ggaagatgaa ctgcagcgta      540
tcaaactaaa aggcaccatt gacgtgtcaa agctggttac ggggactgtc ctggctgtgt      600
ttggctccgt gagagacgac gggaagtttc tggtggagga ctattgcttt gctgaccttg      660
ctccccagaa gcccgcaccc ccacttgaca cagataggtt tgtgctactg gtgtccggcc      720
tgggcctggg tggcggtgga ggcgagagcc tgctgggcac ccagctgctg gtggatgtgg      780
tgacggggca gcttggggac gaaggggagc agtgcagcgc cgcccacgtc tcccgggtta      840
tcctcgctgg caacctcctc agccacagca cccagagcag ggattctatc aataaggcca      900
aatacctcac caagaaaacc caggcagcca gcgtggaggc tgttaagatg ctggatgaga      960
tcctcctgca gctgagcgcc tcagtgcccg tggacgtgat gccaggcgag tttgatccca     1020
ccaattacac gctcccccag cagcccctcc acccctgcat gttcccgctg gccactgcct     1080
actccacgct ccagctggtc accaacccct accaggccac cattgatgga gtcagatttt     1140
tggggacatc aggacagaac gtgagtgaca ttttccgata cagcagcatg gaggatcact     1200
tggagatcct ggagtggacc ctgcgggtcc gtcacatcag ccccacagcc cctgacactc     1260
taggttgtta ccccttctac aaaactgacc cgttcatctt cccagagtgc ccgcatgtct     1320
acttttgtgg caacaccccc agctttggct ccaaaatcat ccgaggtcct gaggaccaga     1380
cagtgctgtt ggtgactgtc cctgacttca gtgccacgca gaccgcctgc cttgtgaacc     1440
tgcgcagcct ggcctgccag cccatcagct tctcgggctt cggggcagag gacgatgacc     1500
tgggaggcct ggggctgggc ccctgactca aaaaagtggt tttgaccaga gaggcccaga     1560
tggaggctgt tcattccctg cagtgtcggc attgtaaata aagcctgagc acttgctgat     1620
gcgagccttg aaaaaaaaaa aaaaaaaa                                        1648
```

<210>  23
<211>  469
<212>  PRT
<213>  Homo sapiens

<400>  23

```
Met Phe Ser Glu Gln Ala Ala Gln Arg Ala His Thr Leu Leu Ser Pro
1               5                   10                  15

Pro Ser Ala Asn Asn Ala Thr Phe Ala Arg Val Pro Val Ala Thr Tyr
            20                  25                  30

Thr Asn Ser Ser Gln Pro Phe Arg Leu Gly Glu Arg Ser Phe Ser Arg
        35                  40                  45
```

```
Gln Tyr Ala His Ile Tyr Ala Thr Arg Leu Ile Gln Met Arg Pro Phe
    50                  55                  60

Leu Glu Asn Arg Ala Gln Gln His Trp Gly Ser Gly Val Gly Val Lys
65                  70                  75                  80

Lys Leu Cys Glu Leu Gln Pro Glu Glu Lys Cys Cys Val Val Gly Thr
                85                  90                  95

Leu Phe Lys Ala Met Pro Leu Gln Pro Ser Ile Leu Arg Glu Val Ser
            100                 105                 110

Glu Glu His Asn Leu Leu Pro Gln Pro Pro Arg Ser Lys Tyr Ile His
        115                 120                 125

Pro Asp Asp Glu Leu Val Leu Glu Asp Glu Leu Gln Arg Ile Lys Leu
    130                 135                 140

Lys Gly Thr Ile Asp Val Ser Lys Leu Val Thr Gly Thr Val Leu Ala
145                 150                 155                 160

Val Phe Gly Ser Val Arg Asp Asp Gly Lys Phe Leu Val Glu Asp Tyr
                165                 170                 175

Cys Phe Ala Asp Leu Ala Pro Gln Lys Pro Ala Pro Pro Leu Asp Thr
            180                 185                 190

Asp Arg Phe Val Leu Leu Val Ser Gly Leu Gly Leu Gly Gly Gly Gly
        195                 200                 205

Gly Glu Ser Leu Leu Gly Thr Gln Leu Leu Val Asp Val Val Thr Gly
    210                 215                 220

Gln Leu Gly Asp Glu Gly Glu Gln Cys Ser Ala Ala His Val Ser Arg
225                 230                 235                 240

Val Ile Leu Ala Gly Asn Leu Leu Ser His Ser Thr Gln Ser Arg Asp
                245                 250                 255

Ser Ile Asn Lys Ala Lys Tyr Leu Thr Lys Lys Thr Gln Ala Ala Ser
            260                 265                 270

Val Glu Ala Val Lys Met Leu Asp Glu Ile Leu Leu Gln Leu Ser Ala
        275                 280                 285

Ser Val Pro Val Asp Val Met Pro Gly Glu Phe Asp Pro Thr Asn Tyr
    290                 295                 300
```

Thr Leu Pro Gln Gln Pro Leu His Pro Cys Met Phe Pro Leu Ala Thr
305 310 315 320

Ala Tyr Ser Thr Leu Gln Leu Val Thr Asn Pro Tyr Gln Ala Thr Ile
325 330 335

Asp Gly Val Arg Phe Leu Gly Thr Ser Gly Gln Asn Val Ser Asp Ile
340 345 350

Phe Arg Tyr Ser Ser Met Glu Asp His Leu Glu Ile Leu Glu Trp Thr
355 360 365

Leu Arg Val Arg His Ile Ser Pro Thr Ala Pro Asp Thr Leu Gly Cys
370 375 380

Tyr Pro Phe Tyr Lys Thr Asp Pro Phe Ile Phe Pro Glu Cys Pro His
385 390 395 400

Val Tyr Phe Cys Gly Asn Thr Pro Ser Phe Gly Ser Lys Ile Ile Arg
405 410 415

Gly Pro Glu Asp Gln Thr Val Leu Leu Val Thr Val Pro Asp Phe Ser
420 425 430

Ala Thr Gln Thr Ala Cys Leu Val Asn Leu Arg Ser Leu Ala Cys Gln
435 440 445

Pro Ile Ser Phe Ser Gly Phe Gly Ala Glu Asp Asp Asp Leu Gly Gly
450 455 460

Leu Gly Leu Gly Pro
465

<210> 24
<211> 504
<212> PRT
<213> Homo sapiens

<400> 24

Met Gly Gly Ala Gly Ala Arg Gly Leu Ala Gly Cys Gly Ala Pro Arg
1 5 10 15

Val Asn Leu Leu Gly Leu Gly Glu Ala Val Trp Thr Lys Gln Val Arg
20 25 30

Ser Val Ala Met Phe Ser Glu Gln Ala Ala Gln Arg Ala His Thr Leu
35 40 45

```
Leu Ser Pro Pro Ser Ala Asn Asn Ala Thr Phe Ala Arg Val Pro Val
    50                  55                  60

Ala Thr Tyr Thr Asn Ser Ser Gln Pro Phe Arg Leu Gly Glu Arg Ser
65                  70                  75                  80

Phe Ser Arg Gln Tyr Ala His Ile Tyr Ala Thr Arg Leu Ile Gln Met
                85                  90                  95

Arg Pro Phe Leu Glu Asn Arg Ala Gln Gln His Trp Gly Ser Gly Val
            100                 105                 110

Gly Val Lys Lys Leu Cys Glu Leu Gln Pro Glu Glu Lys Cys Cys Val
        115                 120                 125

Val Gly Thr Leu Phe Lys Ala Met Pro Leu Gln Pro Ser Ile Leu Arg
    130                 135                 140

Glu Val Ser Glu Glu His Asn Leu Leu Pro Gln Pro Pro Arg Ser Lys
145                 150                 155                 160

Tyr Ile His Pro Asp Asp Glu Leu Val Leu Glu Asp Glu Leu Gln Arg
                165                 170                 175

Ile Lys Leu Lys Gly Thr Ile Asp Val Ser Lys Leu Val Thr Gly Thr
            180                 185                 190

Val Leu Ala Val Phe Gly Ser Val Arg Asp Asp Gly Lys Phe Leu Val
        195                 200                 205

Glu Asp Tyr Cys Phe Ala Asp Leu Ala Pro Gln Lys Pro Ala Pro Pro
    210                 215                 220

Leu Asp Thr Asp Arg Phe Val Leu Leu Val Ser Gly Leu Gly Leu Gly
225                 230                 235                 240

Gly Gly Gly Gly Glu Ser Leu Leu Gly Thr Gln Leu Leu Val Asp Val
                245                 250                 255

Val Thr Gly Gln Leu Gly Asp Glu Gly Glu Gln Cys Ser Ala Ala His
            260                 265                 270

Val Ser Arg Val Ile Leu Ala Gly Asn Leu Leu Ser His Ser Thr Gln
        275                 280                 285

Ser Arg Asp Ser Ile Asn Lys Ala Lys Tyr Leu Thr Lys Lys Thr Gln
    290                 295                 300
```

```
Ala Ala Ser Val Glu Ala Val Lys Met Leu Asp Glu Ile Leu Leu Gln
305                 310                 315                 320

Leu Ser Ala Ser Val Pro Val Asp Val Met Pro Gly Glu Phe Asp Pro
                325                 330                 335

Thr Asn Tyr Thr Leu Pro Gln Gln Pro Leu His Pro Cys Met Phe Pro
            340                 345                 350

Leu Ala Thr Ala Tyr Ser Thr Leu Gln Leu Val Thr Asn Pro Tyr Gln
        355                 360                 365

Ala Thr Ile Asp Gly Val Arg Phe Leu Gly Thr Ser Gly Gln Asn Val
    370                 375                 380

Ser Asp Ile Phe Arg Tyr Ser Ser Met Glu Asp His Leu Glu Ile Leu
385                 390                 395                 400

Glu Trp Thr Leu Arg Val Arg His Ile Ser Pro Thr Ala Pro Asp Thr
                405                 410                 415

Leu Gly Cys Tyr Pro Phe Tyr Lys Thr Asp Pro Phe Ile Phe Pro Glu
            420                 425                 430

Cys Pro His Val Tyr Phe Cys Gly Asn Thr Pro Ser Phe Gly Ser Lys
        435                 440                 445

Ile Ile Arg Gly Pro Glu Asp Gln Thr Val Leu Leu Val Thr Val Pro
    450                 455                 460

Asp Phe Ser Ala Thr Gln Thr Ala Cys Leu Val Asn Leu Arg Ser Leu
465                 470                 475                 480

Ala Cys Gln Pro Ile Ser Phe Ser Gly Phe Gly Ala Glu Asp Asp Asp
                485                 490                 495

Leu Gly Gly Leu Gly Leu Gly Pro
            500
```

**Description**

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of one or more DNA repair genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of one or more DNA repair genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of one or more, for example, 1, 2, 3, 4 or all, DNA repair genes selected from the group consisting of: APTX, BRCA2, NUDT1, PMS2, and POLD2, said gene expression profile(s) being determined in a biological sample obtained from the subject,
- determining the prediction of the radiotherapy response based on the gene expression profile(s) for the one or more DNA repair genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated.

The present invention is based on the idea that, since the status of the immune system and of the immune microenvironment have an impact on therapy effectiveness, the ability to identify markers predictive for this effect might help to be better able to predict overall RT response.

DNA repair system has evolved to maintain the genomic integrity to defend against both endogenous and exogenous sources of DNA damage, such as endogenous factors include reactive oxygen species, replication errors or mistakes in meiosis and exogenous factors include ultraviolet (UV) radiation, ionizing radiation (IR), and some other chemicals or chemotherapeutic agents (see Kang Z. et al., "DNA repair in cancer", J Oncol, Vol. 2019, Article 8676947). Multiple repair pathways (direct repair, base excision repair, nucleotide excision repair, mismatch repair, nonhomologous end joining, and homologous recombination pathways) can be aroused from the diverse forms of DNA lesions including mismatch paired bases, small deletions or insertions, and DNA single or double-strand breaks. These repair pathways also exert crosstalk with others to complete the whole DNA repair process.

The deficient DNA repair causing prolonged existence of DNA damages can lead to genes mutations, chromosome rearrangements, genomic instability, and finally carcinogenesis. Indeed, defects in DNA repair pathways contribute to many heritable cancer predisposition syndromes; however, cancer-related DNA repair deficiency may also occur in sporadic cancer case. Defective DNA repair is common in carcinogenesis and plays a critical role in cancer progression. For example, genetic mutations in DNA mismatch repair genes are involved in reducing mismatch repair and increasing the risk to colon and uterine tumors; BRCA1, BRCA2, and PALB2 genes mutations result in defective homologous recombination repair and are associated with the carcinogenesis of breast and ovarian cancer. In these years, many cancer-related germline mutations in DNA repair have been reported; thus to detect these genetic variations gives us a chance to evaluate the cancer risk of the individual with these mutations.

Furthermore, these defects in DNA repair pathways may have therapeutic implications for clinical practice. Most recently, a series of therapeutic strategy have been exploited, such as platinum chemotherapies and PARP inhibitors in homologous recombination defected breast and ovarian cancers and inhibitors of immune checkpoints CTLA-4, PD1/PD-L1 in the case of mismatch repair deficiency cancers. These observations give the direction for further research to investigate the defects in DNA repair pathways that may serve as very useful biomarkers for the choice of suitable oncotherapy.

The identified DNA repair genes APTX, BRCA2, NUDT1, PMS2, and POLD2 were identified as follows: A group of 538 prostate cancer patients were treated with RP and the prostate cancer tissue was stored. A number of these patients experienced biochemical recurrence and was treated with SRT. For 151 of these patients, the RNA expressed in the originally stored prostate cancer tissue was analysed using RNA sequencing. The mRNA expression of DNA repair related genes was compared for the 26 out of 151 patients that died due to prostate cancer, versus the 125 out of 151 patients who survived. For the five molecules APTX, BRCA2, NUDT1, PMS2, and POLD2, the expression level was significantly different for the survivors, suggesting that they have value in the prediction of survival after SRT. Several of these five molecules were found to correlate with prostate cancer, be it in different settings, as described further below.

The term "APTX" refers to the Aprataxin gene (Ensembl: ENSG00000137074), for example, to the sequence as defined in NCBI Reference Sequence NM_175073 or in NCBI Reference Sequence NM_001195254 or in NCBI Reference Sequence NM_175069 or in NCBI Reference Sequence NM_001195250, specifically, to the nucleotide sequences as set forth in SEQ ID NO: 1 or in SEQ ID NO:2 or in SEQ ID NO:3 or in SEQ ID NO:4, which correspond to the sequences of the above indicated NCBI Reference Sequences of the APTX transcript, and also relates to the corresponding amino acid sequences for example as set forth in SEQ ID NO:5 or in SEQ ID NO:6 or in SEQ ID NO:7 or in SEQ ID NO:8, which correspond to the protein sequences defined in NCBI Protein Accession Reference Sequence NP_778243 and in NCBI Protein Accession Reference Sequence NP_001182183 and in NCBI Protein Accession Reference Sequence NP_778239 and in NCBI Protein Accession Reference Sequence NP_001182179 encoding the APTX polypeptide.

The term "APTX" also comprises nucleotide sequences showing a high degree of homology to APTX, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2 or in SEQ ID NO:3 or in SEQ ID NO:4 or amino acid sequences being at least 75%, 80%, 85%, 90%, 91%, 92%, 93%, 94%, 95%, 96%, 97%, 98% or 99% identical to the sequence as set forth in SEQ ID NO:5 or in SEQ ID NO:6 or in SEQ ID NO:7 or in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:5 or in SEQ ID NO:6 or in SEQ ID NO:7 or in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2 or in SEQ ID NO:3 or in SEQ ID NO:4.

The term "BRAC2" refers to the BRAC2 DNA Repair Associated gene (Ensembl: ENSG00000139618), for example, to the sequence as defined in NCBI Reference Sequence NM_000059, specifically, to the nucleotide sequence as set forth in SEQ ID NO:9, which corresponds to the sequence of the above indicated NCBI Reference Sequence of the BRAC2 transcript, and also relates to the corresponding amino acid sequence for example as set forth in SEQ ID NO:10, which corresponds to the protein sequence defined in NCBI Protein Accession Reference Sequence NP_000050 encoding the BRAC2 polypeptide.

The term "BRAC2" also comprises nucleotide sequences showing a high degree of homology to BRAC2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO: 10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "NUDT1" refers to the Nudix Hydrolase 1 gene (Ensembl: ENSG00000106268), for example, to the sequence as defined in NCBI Reference Sequence NM_198954 or in NCBI Reference Sequence NM_198953, specifically, to the nucleotide sequences as set forth in SEQ ID NO:11 or in SEQ ID NO: 12, which correspond to the sequences of the above indicated NCBI Reference Sequences of the NUDT1 transcript, and also relates to the corresponding amino acid sequences for example as set forth in SEQ ID NO: 13 or in SEQ ID NO: 14, which correspond to the protein sequences defined in NCBI Protein Accession Reference Sequence NP_945192 and in NCBI Protein Accession Reference Sequence NP_945191 encoding the NUDT1 polypeptide.

The term "NUDT1" also comprises nucleotide sequences showing a high degree of homology to NUDT1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO: 12 or amino acid sequences being at least 75%, 80%, 85%, 90%, 91%, 92%, 93%, 94%, 95%, 96%, 97%, 98% or 99% identical to the sequence as set forth in SEQ ID NO: 13 or in SEQ ID NO: 14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:13 or in SEQ ID NO:14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO: 12.

The term "PMS2" refers to the PMS1 Homolog 2, Mismatch Repair System Component gene (Ensembl: ENSG00000122512), for example, to the sequence as defined in NCBI Reference Sequence NM_000535 or in NCBI Reference Sequence NM_001322005 or in NCBI Reference Sequence NM_001322008, specifically, to the nucleotide sequences as set forth in SEQ ID NO: 15 or in SEQ ID NO: 16 or in SEQ ID NO: 17, which correspond to the sequences of the above indicated NCBI Reference Sequences of the PMS2 transcript, and also relates to the corresponding amino acid sequences for example as set forth in SEQ ID NO: 18 or in SEQ ID NO: 19 or in SEQ ID NO:20, which correspond to the protein sequences defined in NCBI Protein Accession Reference Sequence NP_000526 and in NCBI Protein Accession Reference Sequence NP_001308934 and in NCBI Protein Accession Reference Sequence NP_001308937 encoding the PMS2 polypeptide.

The term "PMS2" also comprises nucleotide sequences showing a high degree of homology to PMS2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:15 or in SEQ ID NO:16 or in SEQ ID NO:17 or amino acid sequences being at least 75%, 80%, 85%, 90%, 91%, 92%, 93%, 94%, 95%, 96%, 97%, 98% or 99% identical to the sequence as set forth in SEQ ID NO: 18 or in SEQ ID NO: 19 or in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 18 or in SEQ ID NO:19 or in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:15 or in SEQ ID NO: 16 or in SEQ ID NO: 17.

The term "POLD2" refers to the DNA Polymerase Delta 2, Accessory Subunit gene (Ensembl: ENSG00000106628), for example, to the sequence as defined in NCBI Reference Sequence NM_001127218 or in NCBI Reference Sequence NM_006230, specifically, to the nucleotide sequences as set forth in SEQ ID NO:21 or in SEQ ID NO:22, which correspond to the sequences of the above indicated NCBI Reference Sequences of the POLD2 transcript, and also relates to the corresponding amino acid sequences for example as set forth in SEQ ID NO:23 or in SEQ ID NO:24, which correspond to the protein sequences defined in NCBI Protein Accession Reference Sequence NP_001120690 and in NCBI Protein Accession Reference Sequence NP_006221 encoding the POLD2 polypeptide.

The term "POLD2" also comprises nucleotide sequences showing a high degree of homology to POLD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22 or amino acid sequences being at least 75%, 80%, 85%, 90%, 91%, 92%, 93%, 94%, 95%, 96%, 97%, 98% or 99% identical to the sequence as set forth in SEQ ID NO:23 or in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:23 or in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the one or more DNA repair genes comprise three or more, preferably, all of the DNA repair genes.

It is preferred that the determining of the prediction of the radiotherapy response comprises combining the gene expression profiles for two or more, for example, 2, 3, 4 or all, of the DNA repair genes with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t)=H_0(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while $H(t)$ is the hazard at any time t. The hazard ratio (or the risk to reach the event) is represented by $\mathrm{Ln}[H(t)/H_0(t)]= w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the prediction of the radiotherapy response is determined as follows:

$$(w_1 \cdot \mathrm{APTX}) + (w_2 \cdot \mathrm{BRCA2}) + (w_3 \cdot \mathrm{NUDT1}) + (w_4 \cdot \mathrm{PMS2}) + (w_5 \cdot \mathrm{POLD2}) \quad (1)$$

where $w_1$ to $w_5$ are weights and APTX, BRCA2, NUDT1, PMS2, and POLD2 are the expression levels of the DNA repair genes.

In one example, $w_1$ may be about -1.0 to 0.0, such as -0.4783, $w_2$ may be about 0.5 to 1.5, such as 1.0455, $w_3$ may be about 0.5 to 1.5, such as 1.1879, $w_4$ may be about -2.0 to -1.0, such as -1.5361, and $w_5$ may be about -2.0 to -1.0, such as -1.7216.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to $<0.1$ or from 0.1 to $<0.25$ or from 0.25 to $<0.5$ or from 0.5 to 1.0 or the like.

It is further preferred that the determining of the prediction of the radiotherapy response is further based on one or more clinical parameters obtained from the subject.

As mentioned above, various measures based on clinical parameters have been investigated. By further basing the prediction of the radiotherapy response on such clinical parameter(s), it can be possible to further improve the prediction.

It is preferred that the one or more clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

It is further preferred that the determining of the prediction of the radiotherapy response comprises combining the gene expression profile(s) for the one or more DNA repair genes and the one or more clinical parameters obtained from the subject with a regression function that had been derived from a population of prostate cancer subjects.

In one particular realization, the prediction of the radiotherapy response is determined as follows:

$$(w_6 \cdot \mathrm{DNArep_model}) + (w_7 \cdot \mathrm{pGGG}) \quad (2)$$

where $w_6$ and $w_7$ are weights, DNArep_model is the above-described regression model based on the expression profiles for the two or more, for example, 2, 3, 4 or all, of the DNA repair genes, and pGGG is the pathological Gleason grade group.

In one example, $w_6$ may be about 0.5 to 1.5, such as 0.9015, and $w_7$ may be about 0.5 to 1.5, such as 0.7818.

It is preferred that the biological sample is obtained from the subject before the start of the radiotherapy. The gene expression profile(s) may be determined in the form of mRNA or protein in tissue of prostate cancer. Alternatively, if the DNA repair genes are present in a soluble form, the gene expression profile(s) may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of one or more, for example, 1, 2, 3, 4 or all, DNA repair genes selected from the group consisting of: APTX, BRCA2, NUDT1, PMS2, and POLD2, said gene expression profile(s) being determined in a biological sample obtained from the subject,
- a processor adapted to determine the prediction of the radiotherapy response based on the gene expression profile(s) for the one or more DNA repair genes, and
- optionally, a providing unit adapted to provide the prediction or a therapy recommendation based on the prediction to a medical caregiver or the subject. In a further aspect of the present invention, a computer program product is presented comprising instructions which, when the program is executed by a computer, cause the computer to carry out a method comprising:
- receiving data indicative of a gene expression profile for each of one or more, for example, 1, 2, 3, 4 or all, DNA repair genes selected from the group consisting of: APTX, BRCA2, NUDT1, PMS2, and POLD2, said gene expression profile(s) being determined in a biological sample obtained from a prostate cancer subject,
- determining the prediction of the radiotherapy response based on the gene expression profile(s) for the one or more DNA repair genes, and
- optionally, providing the prediction or a therapy recommendation based on the prediction to a medical caregiver or the subject.

In a further aspect of the present invention, a diagnostic kit is presented, comprising:

- at least one primer and/or probe for determining the gene expression profile for each of one or more, for example, 1, 2, 3, 4 or all, DNA repair genes selected from the group consisting of: APTX, BRCA2, NUDT1, PMS2, and POLD2, in a biological sample obtained from the subject, and
- optionally, an apparatus as defined in claim 10 or a computer program product as defined in claim 11.

In a further aspect of the present invention, a use of the kit as defined in claim 12 is presented.

It is preferred that the use as defined in claim 13 is in a method of predicting a response of a prostate cancer subject to radiotherapy.

In a further aspect of the present invention, a method is presented, comprising:

- receiving a biological sample obtained from a prostate cancer subject,
- using the kit as defined in claim 12 to determine a gene expression profile for each of one or more, for example, 1, 2, 3, 4 or all, DNA repair genes selected from the group consisting of: APTX, BRCA2, NUDT1, PMS2, and POLD2, in the biological sample obtained from the subject.

In a further aspect of the present invention, a use of a gene expression profile for each of one or more, for example, 1, 2, 3, 4 or all, DNA repair genes selected from the group consisting of: APTX, BRCA2, NUDT1, PMS2, and POLD2, in a method of predicting a response of a prostate cancer subject to radiotherapy is presented, comprising:

- determining the prediction of the radiotherapy response based on the gene expression profile(s) for the one or more DNA repair genes, and
- optionally, providing the prediction or a therapy recommendation based on the prediction to a medical caregiver or the subject.

It shall be understood that the method of claim 1, the apparatus of claim 10, the computer program product of claim 11, the diagnostic kit of claim 12, the use of the diagnostic kit of claim 13, the method of claim 15, and the use of a gene expression profile(s) of claim 16 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy.

Fig. 2 shows a ROC curve analysis of two predictive models.

Fig. 3 shows a Kaplan-Meier curve analysis of the DNA repair model (DNArep_model). The clinical endpoint that was tested was the time to metastases (TTM) after the start of salvage radiation therapy (SRT) due to post-surgical disease recurrence.

Fig. 4 shows a Kaplan-Meier curve analysis of the DNA repair & pGGG combination model (DNArep&pGGG model). The clinical endpoint that was tested was the time to metastases (TTM) after the start of salvage radiation therapy (SRT) due to post-surgical disease recurrence.

Fig. 5 shows a Kaplan-Meier curve analysis of the DNA repair model (DNArep_model). The clinical endpoint that was tested was the time to prostate cancer specific death (PCa Death) after the start of the salvage radiation therapy (SRT) due to post-surgical disease recurrence.

Fig. 6 shows a Kaplan-Meier curve of the DNA repair & pGGG combination model (DNArep&pGGG model). The clinical endpoint that was tested was the time to prostate cancer specific death (PCa Death) after the start of salvage radiation therapy (SRT) due to post-surgical disease recurrence.

DETAILED DESCRIPTION OF EMBODIMENTS

**Overview Of Radiotherapy Response Prediction**

Fig. 1 shows schematically and exemplarily a flowchart of an embodiment of a method of predicting a response of a prostate cancer subject to radiotherapy. The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile for each of two or more, for example, 2, 3, 4 or all, DNA repair genes selected from the group consisting of: APTX, BRCA2, NUDT1, PMS2, and POLD2, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more DNA repair genes.

At step S106, a regression function for assigning a prediction of the radiotherapy response is determined based on the gene expression profiles for the two or more DNA repair genes, APTX, BRCA2, NUDT1, PMS2, and/or POLD2, obtained for at least some of the biological samples obtained for the first set of patients and respective results obtained from the monitoring. In one particular realization, the regression function is determined as specified in Eq. (1) above.

At step S108, a biological sample is obtained from a patient (subject or individual). The patient can be a new patient or one of the first set.

At step S110, a gene expression profile is obtained for each of the two or more, for example, 2, 3, 4 or all, DNA repair genes, e.g., by performing PCR on the biological sample.

At step S112, a prediction of the radiotherapy response based on the gene expression profiles for the two or more DNA repair genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using two or more primers and/or probes and/or two or more sets thereof.

In one embodiment, steps S104 and S110 further comprise obtaining one or more clinical parameters from the first set of patients and the patient, respectively. The one or more clinical parameters may comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score. The regression function for assigning the prediction of the radiotherapy response that is determined in step S106 is then further based on the one or more clinical parameters obtained from at least some of the first set of patients. In step S112, the prediction of the radiotherapy response is then further based on the one or more clinical parameters, e.g., the pathological Gleason grade group (pGGG), obtained from the patient and is determined for the patient using the regression function.

The immune system interacts in a strong manner with prostate cancer, both on a systemic level and in the tumour microenvironment. DNA repair genes play a central role in the regulation of immune activity. DNA repair genes may therefore provide information on the effectiveness of RT. However, which DNA repair genes may have predictive value in this application is extremely difficult to deduce from existing literature due to the many factors that influence the exact function of DNA repair genes.

We investigated the extent to which the expression of DNA repair genes in prostate cancer tissue correlates with the recurrence of disease after radical RT or SRT.

We have identified five members of the family of DNA repair genes for which the degree of expression in prostate cancer tissue significantly correlates with mortality after SRT, in a cohort of 151 prostate cancer patients (TABLE 1).

Based on the significant correlation with outcome after RT, we expect that the identified molecules will provide predictive value with regard to the effectiveness of radical RT and/or SRT.

TABLE 1: Univariate Cox regression analysis in a cohort with 151 prostate cancer patients. The tested endpoints in the patient cohort were a) post-treatment metastases progression free survival, and b) post-treatment disease specific survival for men undergoing salvage radiation (SRT) after post-surgical disease recurrence. The number of events and percentage per endpoint is indicated in parenthesis. The table indicates for each tested DNA repair gene the association in terms of risk (Hazard Ratio) and significance (p-value) to the tested endpoint.

| **Data Set** | **(Prostate RNAseq)** | | | |
|---|---|---|---|---|
| **# Patients** | **151** | | | |
| **Outcome** | **Post-Salvage-Radiation Outcome** | | | |
| **Endpoint (# events / # patients; % events)** | **Metastases (#65/#151; 43.0%)** | | **Prostate Cancer Mortality (#26/#151; 17.2%)** | |
| **DNA repair** | **p-value** | **HR** | **p-value** | **HR** |
| **APTX** | 0.024 | 0.31 | 0.024 | 0.33 |
| **BRCA2** | 0.013 | 1.86 | 0.006 | 1.91 |
| **NUDT1** | 0.18 | 1.83 | 0.02 | 2.35 |
| **PMS2** | 0.02 | 0.26 | 0.01 | 0.23 |
| **POLD2** | 0.002 | 0.09 | 0.01 | 0.16 |

RESULTS

**Cox Regression Analysis**

We then set out to test whether the combination of these five DNA repair genes will exhibit more prognostic value. With Cox regression we modelled the expression levels of the five DNA repair genes to prostate cancer specific death after post-surgical salvage RT either with (DNArep&pGGG_model) or without (DNArepmodel) the presence of the variable pathological Gleason grade group (pGGG). We tested the two models in ROC curve analysis as well as in Kaplan-Meier survival analysis.

The Cox regression functions were derived as follows:

DNArep_model:

$$(w_1 \cdot \text{APTX}) + (w_2 \cdot \text{BRCA2}) + (w_3 \cdot \text{NUDT1}) + (w_4 \cdot \text{PMS2}) + (w_5 \cdot \text{POLD2})$$

DNArep&pGGG_model:

$$(w_6 \cdot \text{DNArep_model}) + (w_7 \cdot \text{pGGG})$$

The details for the weights $w_1$ to $w_7$ are shown in the following TABLE 3.

TABLE 3: Variables and weights for the two Cox regression models, i.e., the DNA Repair model (DNArep model) and the DNA Repair & pGGG combination model (DNArep&pGGG_model); NA - not available.

| **Variable** | **Weights** | | |
|---|---|---|---|
| **Model** | | **DNArep_model** | **DNArep&pGGG_model** |
| **APTX** | $w_1$ | -0.4783 | NA |
| **BRCA2** | $w_2$ | 1.0455 | NA |
| **NUDT1** | $w_3$ | 1.1879 | NA |
| **PMS2** | $w_4$ | -1.5361 | NA |
| **POLD2** | $w_5$ | -1.7216 | NA |
| **DNArep_model** | $w_6$ | NA | 0.9015 |
| **pGGG** | $w_7$ | NA | 0.7818 |

**ROC Curve Analysis**

Next, we tested the combined Cox regression model as outlined above its power to predict 10-year prostate cancer specific death after start of salvage radiation due to post-surgical disease recurrence. The performance of the model was compared to the clinical risk score CAPRA-S (see Cooperberg M.R. et al., "The CAPRA-S score: A straightforward tool for improved prediction of outcomes after radical prostatectomy", Cancer, Vol. 117, No. 22, pages 5039-5046, 2011).

Fig. 2 shows a ROC curve analysis of two predictive models. The DNArep&pGGG_model (AUC=0.94) is the Cox regression model based on five DNA repair genes and the pathology Gleason grade group (pGGG) information. The CAPRA_S (AUC=0.74) is the clinical CAPRA-S score (Cancer of the Prostate Risk Assessment score).

**Kaplan-Meier Survival Analysis**

For Kaplan-Meier curve analysis, the Cox regression function of the two risk models (DNArep_model and DNArep&pGGG_model) was categorized into two sub-cohorts based on a cut-off (see description of figures below). The goal was to create patient classes by separating the classes with the mean risk score as calculated from the DNArep_model and DNArep&pGGG_model, respectively.

The patient classes represent an increasing risk to experience the tested clinical endpoints of time to development of metastases (Figs. 3 and 4) or time to prostate cancer specific death (Figs. 5 and 6) since the start of salvage RT for the two created risk models (DNArep_model; DNArep&pGGG_model).

Fig. 3 shows a Kaplan-Meier curve analysis of the DNArep_model. The clinical endpoint that was tested was the time to metastases (TTM) after the start of salvage radiation therapy (SRT) due to post-surgical disease recurrence. Patients were stratified into two cohorts (low vs. high) according to their risk to experience the clinical endpoint as predicted by the DNArep regression model using the value -3.37 as cut-off (logrank p<0.0001; HR=3.3; 95% CI=1.9-5.9). The following supplementary list indicate the number of patients at risk for the DNArep_model classes analyzed, i.e., the patients at risk at any time interval +20 months after surgery are shown: Low risk: 60, 61, 59, 57, 51, 45,44, 22, 2, 0; High risk: 66, 50, 44, 41, 33, 22, 19, 7, 1, 0.

Fig. 4 shows a Kaplan-Meier curve analysis of the DNArep&pGGG_model. The clinical endpoint that was tested was the time to metastases (TTM) after the start of salvage radiation therapy (SRT) due to post-surgical disease recurrence. Patients were stratified into two cohorts (low vs. high) according to their risk to experience the clinical endpoint as predicted by the DNArep&pGGG regression model using the value -1.58 as cut-off (logrank p<0.0001; HR=4.6; 95% CI=2.6-8.1). The following supplementary lists indicate the number of patients at risk for the DNArep&pGGG_model classes analyzed, i.e., the patients at risk at any time interval +20 months after surgery are shown: Low risk: 58, 54, 54, 52, 47, 44, 43, 21, 2, 0; High risk: 77, 57, 49, 46, 37, 23, 20, 8, 1, 0.

Fig. 5 shows a Kaplan-Meier curve analysis of the DNArep_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the DNArep regression model using the value -3.37 as cut-off (logrank p<0.0001; HR=7.9; 95% CI=3.6-17.1). The following supplementary list indicate the number of patients at risk for the DNArep_model classes analyzed, i.e., the patients at risk at any time interval +20 months after surgery are shown: Low risk: 74, 74, 68, 63, 56, 48, 44, 24, 3, 1, 0; High risk: 77, 71, 62, 51, 40, 29, 23, 11, 3, 2, 0.

Fig. 6 shows a Kaplan-Meier curve analysis of the DNArep&pGGG_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the DNArep&pGGG regression model using the value -1.58 as cut-off (logrank $p<0.0001$; HR=NA). The following supplementary lists indicate the number of patients at risk for the DNArep&pGGG_model classes analyzed, i.e., the patients at risk at any time interval +20 months after surgery are shown: Low risk: 61, 61, 58, 56, 50, 47, 43, 21, 3, 1, 0; High risk: 90, 84, 72, 58, 46, 30, 24, 14, 3, 2, 0.

For example, the Kaplan-Meier analysis as shown in Figs. 3 to 6 demonstrates the presence of different patient risk groups. The risk group of a patient is determined by the probability to suffer from the respective clinical endpoint (metastases, prostate cancer death) as calculated by the risk model DNArepmodel or DNArep&pGGG model. Depending on the predicted risk of a patient (i.e., depending on in which risk group 1 or 2 the patient may belong) different types of interventions might be indicated. In the low risk category, standard of care (SOC) which is SRT potentially combined with SADT (salvage androgen deprivation therapy) delivers acceptable long-term oncological control. Dose escalation of the applied RT and/or combination with chemotherapy might provide improved longitudinal outcomes, and can also be considered. This is definitely not the case for the patient group with a high risk to experience any of the relevant outcomes. In this patient group, escalation of intervention may be indicated. Options for escalation are early combination of SRT (considering higher dose regimens), SADT, and chemotherapy. Other options are alternative therapies like immunotherapies (e.g., Sipuleucil-T), especially since the risk is defined by expression of DNA repair molecules, or other experimental therapies.

**Discussion**

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinicopathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of one or more, for example, 1, 2, 3, 4 or all, DNA repair genes selected from the group consisting of: APTX, BRCA2, NUDT1, PMS2, and POLD2, said gene expression profile(s) being determined in a biological sample obtained from the subject, determining the prediction of the radiotherapy response based on the gene expression profile(s) for the one or more DNA repair genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. DNA repair genes play a central role in the regulation of immune activity. The identified DNA repair genes were found to exhibit a significant correlation with outcome after RT, wherefore we expect that they will provide predictive value with regard to the effectiveness of radical RT and/or SRT.

**The attached Sequence Listing, entitled 2020PF00060_Sequence Listing_ST25 is incorporated herein by reference, in its entirety.**

## Claims

**1.** A method of predicting a response of a prostate cancer subject to radiotherapy, comprising:

- determining or receiving the result of a determination of a gene expression profile for each of one or more, for example, 1, 2, 3, 4 or all, DNA repair genes selected from the group consisting of: APTX, BRCA2, NUDT1, PMS2, and POLD2, said gene expression profile(s) being determined in a biological sample obtained from the subject,
- determining the prediction of the radiotherapy response based on the gene expression profile(s) for the one or more DNA repair genes, and
- optionally, providing the prediction or a therapy recommendation based on the prediction to a medical caregiver or the subject.

**2.** The method as defined in claim 1, wherein the one or more DNA repair genes comprise three or more, preferably, all of the DNA repair genes.

**3.** The method as defined in claim 1 or 2, wherein the determining of the prediction of the radiotherapy response comprises combining the gene expression profiles for two or more, for example, 2, 3, 4 or all, of the DNA repair genes with a regression function that had been derived from a population of prostate cancer subjects.

**4.** The method as defined in claim 1 or 2, wherein the determining of the prediction of the radiotherapy response is further based on one or more clinical parameters obtained from the subject.

**5.** The method as defined in claim 4, wherein the one or more clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

**6.** The method as defined in claim 4 or 5, wherein the determining of the prediction of the radiotherapy response comprises combining the gene expression profile(s) for the one or more DNA repair genes and the one or more clinical parameters obtained from the subject with a regression function that had been derived from a population of prostate cancer subjects.

**7.** The method as defined in any of claims 1 to 6, wherein the biological sample is obtained from the subject before the start of the radiotherapy.

**8.** The method as defined in any of claims 1 to 7, wherein the radiotherapy is radical radiotherapy or salvage radiotherapy.

**9.** The method as defined in any of claims 1 to 8, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

**10.** An apparatus for predicting a response of a prostate cancer subject to radiotherapy, comprising:

- an input adapted to receive data indicative of a gene expression profile for each of one or more, for example, 1, 2, 3, 4 or all, DNA repair genes selected from the group consisting of: APTX, BRCA2, NUDT1, PMS2, and POLD2, said gene expression profile(s) being determined in a biological sample obtained from the subject,
- a processor adapted to determine the prediction of the radiotherapy response based on the gene expression profile(s) for the one or more DNA repair genes, and
- optionally, a providing unit adapted to provide the prediction or a therapy recommendation based on the prediction to a medical caregiver or the subject.

**11.** A computer program product comprising instructions which, when the program is executed by a computer, cause the computer to carry out a method comprising:

- receiving data indicative of a gene expression profile for each of one or more, for example, 1, 2, 3, 4 or all, DNA repair genes selected from the group consisting of: APTX, BRCA2, NUDT1, PMS2, and POLD2, said gene expression profile(s) being determined in a biological sample obtained from a prostate cancer subject,
- determining the prediction of the radiotherapy response based on the gene expression profile(s) for the one or more DNA repair genes, and
- optionally, providing the prediction or a therapy recommendation based on the prediction to a medical caregiver or the subject.

**12.** A diagnostic kit, comprising:

- at least one primer and/or probe for determining the gene expression profile for each of one or more, for example, 1, 2, 3, 4 or all, DNA repair genes selected from the group consisting of: APTX, BRCA2, NUDT1, PMS2, and POLD2, in a biological sample obtained from the subject, and
- optionally, an apparatus as defined in claim 10 or a computer program product as defined in claim 11.

**13.** Use of the kit as defined in claim 12.

**14.** The use as defined in claim 13 in a method of predicting a response of a prostate cancer subject to radiotherapy.

**15.** A method, comprising:

- receiving a biological sample obtained from a prostate cancer subject,
- using the kit as defined in claim 12 to determine a gene expression profile for each of one or more, for example, 1, 2, 3, 4 or all, DNA repair genes selected from the group consisting of: APTX, BRCA2, NUDT1, PMS2, and POLD2, in the biological sample obtained from the subject.

**16.** Use of a gene expression profile for each of one or more, for example, 1, 2, 3, 4 or all, DNA repair genes selected from the group consisting of: APTX, BRCA2, NUDT1, PMS2, and POLD2, in a method of predicting a response of a prostate cancer subject to radiotherapy, comprising:

- determining the prediction of the radiotherapy response based on the gene expression profile(s) for the one or more DNA repair genes, and
- optionally, providing the prediction or a therapy recommendation based on the prediction to a medical caregiver or the subject.

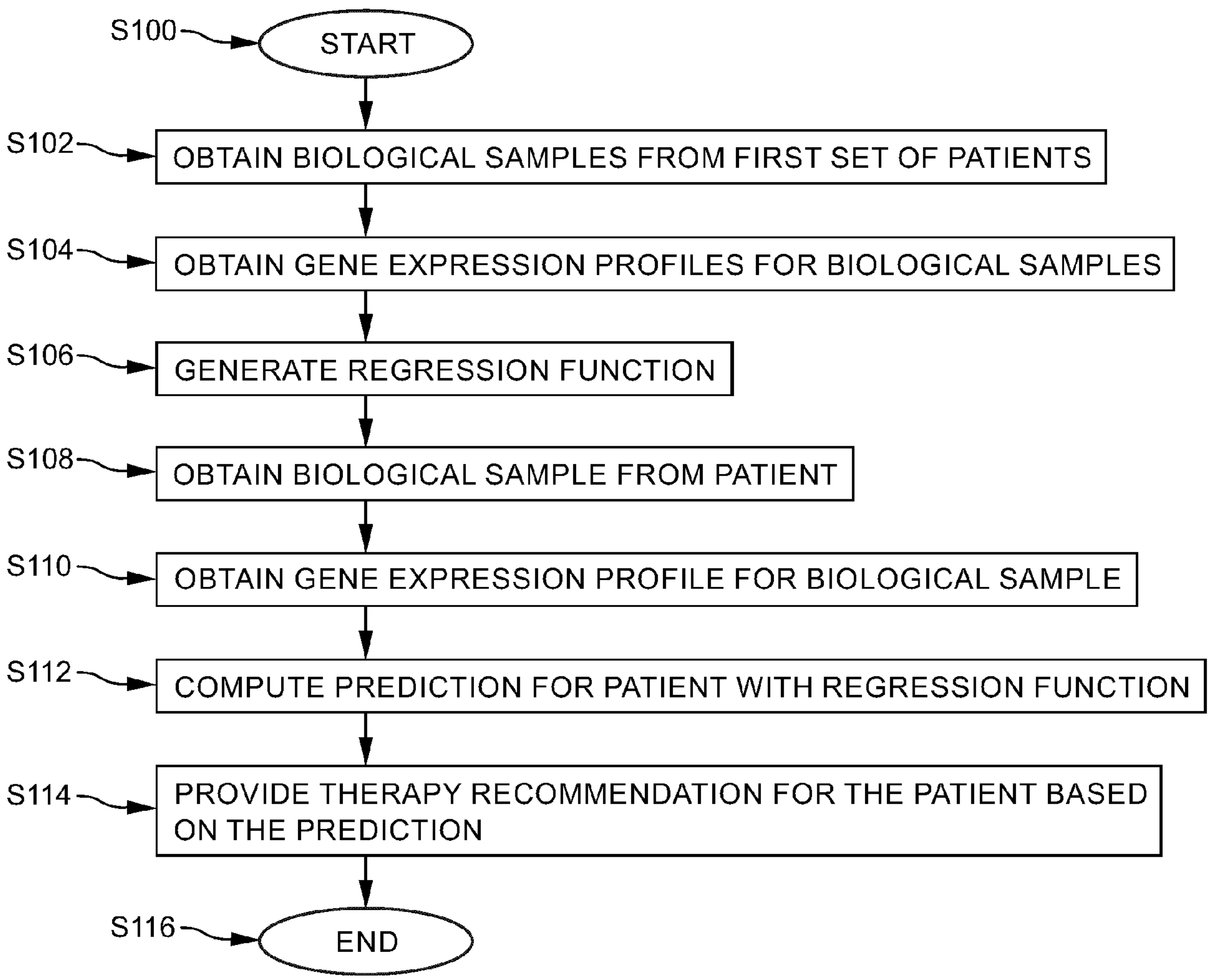
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION FUNCTION
S108
OBTAIN BIOLOGICAL SAMPLE FROM PATIENT
S110
OBTAIN GENE EXPRESSION PROFILE FOR BIOLOGICAL SAMPLE
S112
COMPUTE PREDICTION FOR PATIENT WITH REGRESSION FUNCTION
S114
PROVIDE THERAPY RECOMMENDATION FOR THE PATIENT BASED ON THE PREDICTION
S116
END

FIG. 1

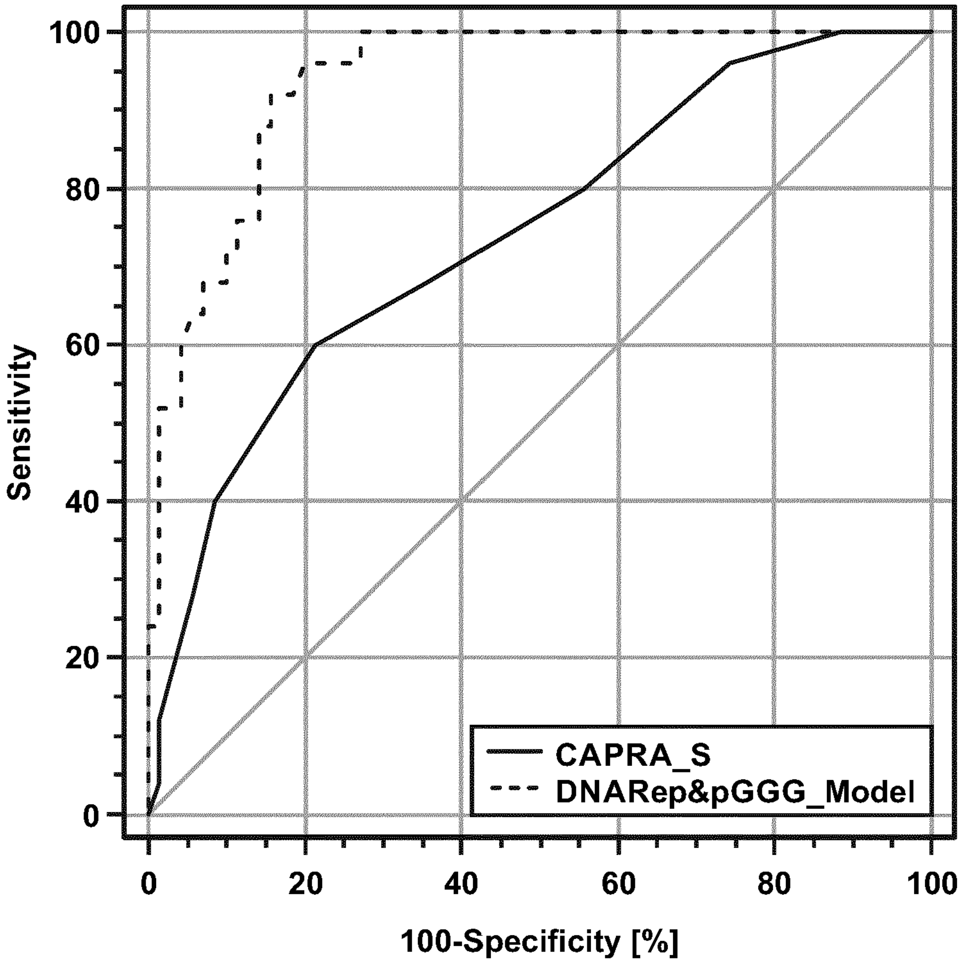
100
80
60
40
20
0
Sensitivity
0
20
40
60
80
100
100-Specificity [%]
CAPRA_S
DNARep&pGGG_Model

FIG. 2

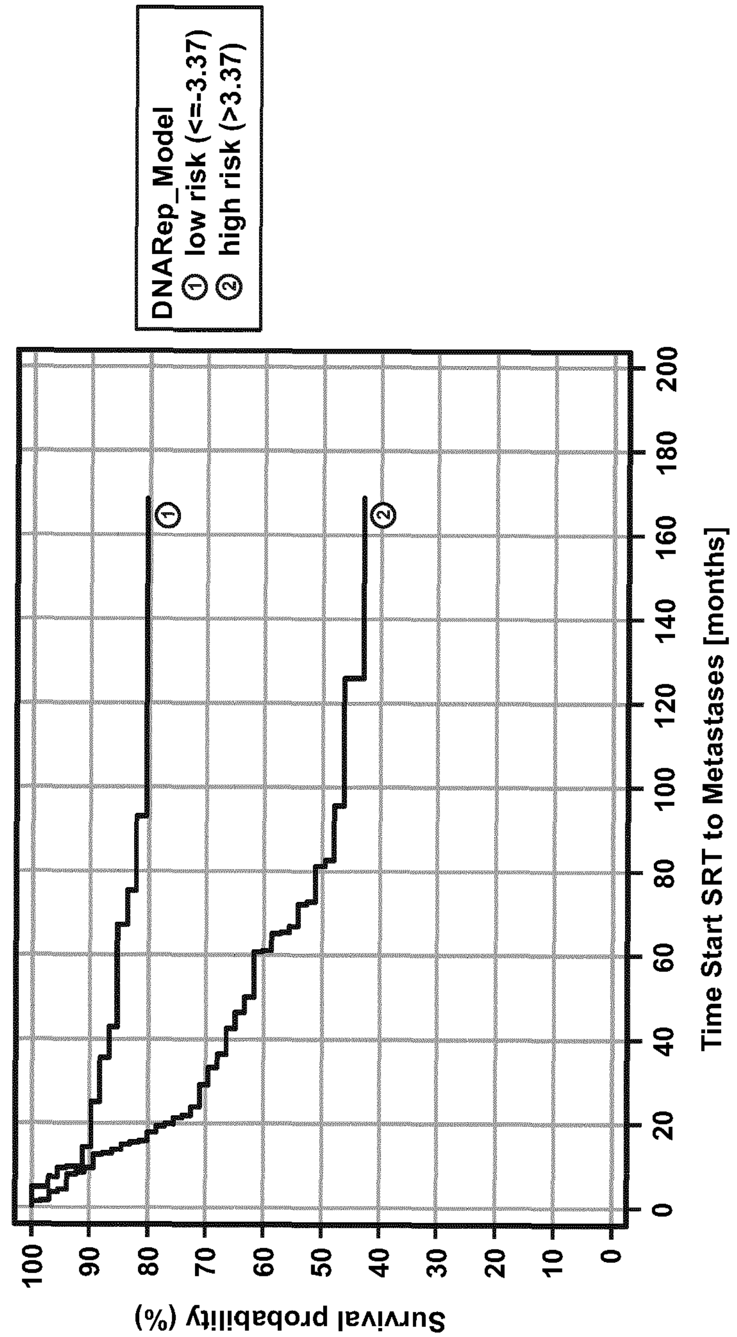
DNARep_Model
① low risk (<=-3.37)
② high risk (>3.37)
Survival probability (%)
100
90
80
70
60
50
40
30
20
10
0
0
20
40
60
80
100
120
140
160
180
200
Time Start SRT to Metastases [months]
①
②

FIG. 3

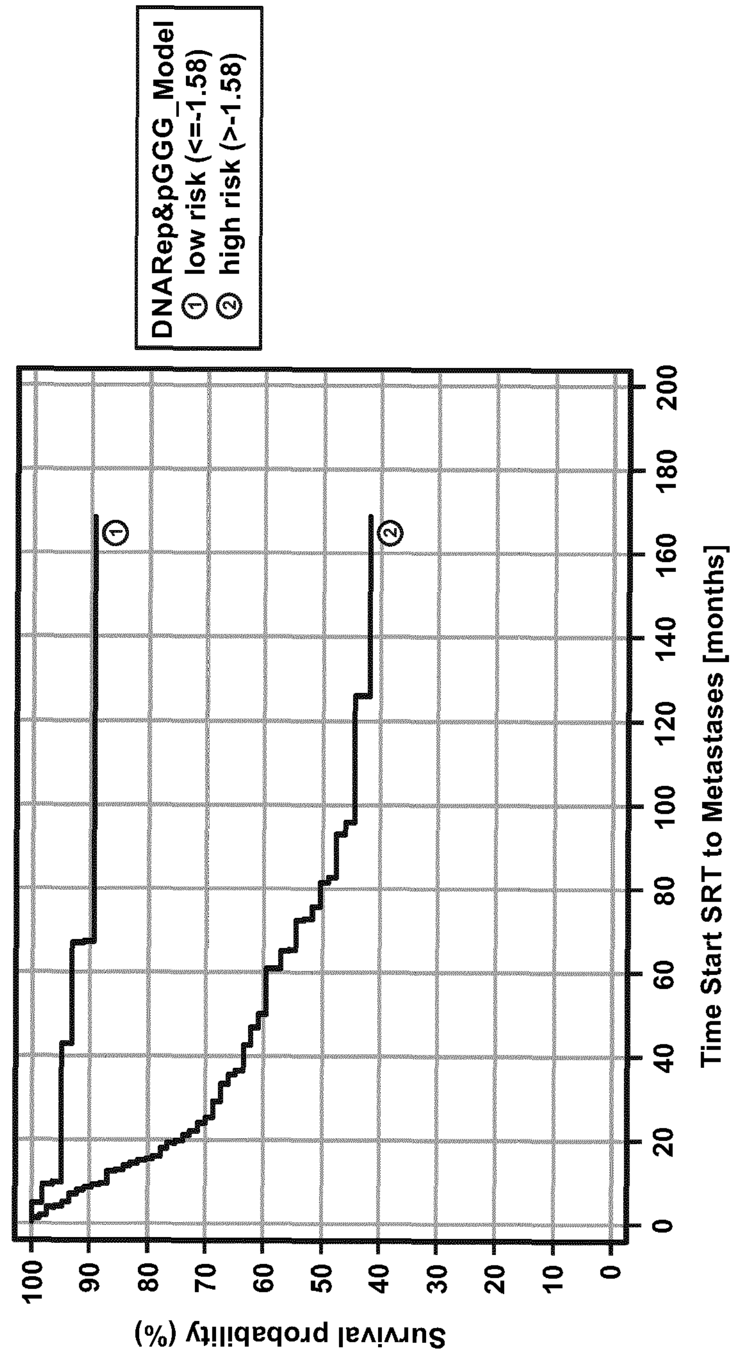
DNARep&pGGG_Model
① low risk (<=-1.58)
② high risk (>-1.58)
Survival probability (%)
100
90
80
70
60
50
40
30
20
10
0
Time Start SRT to Metastases [months]
0
20
40
60
80
100
120
140
160
180
200
①
②

FIG. 4

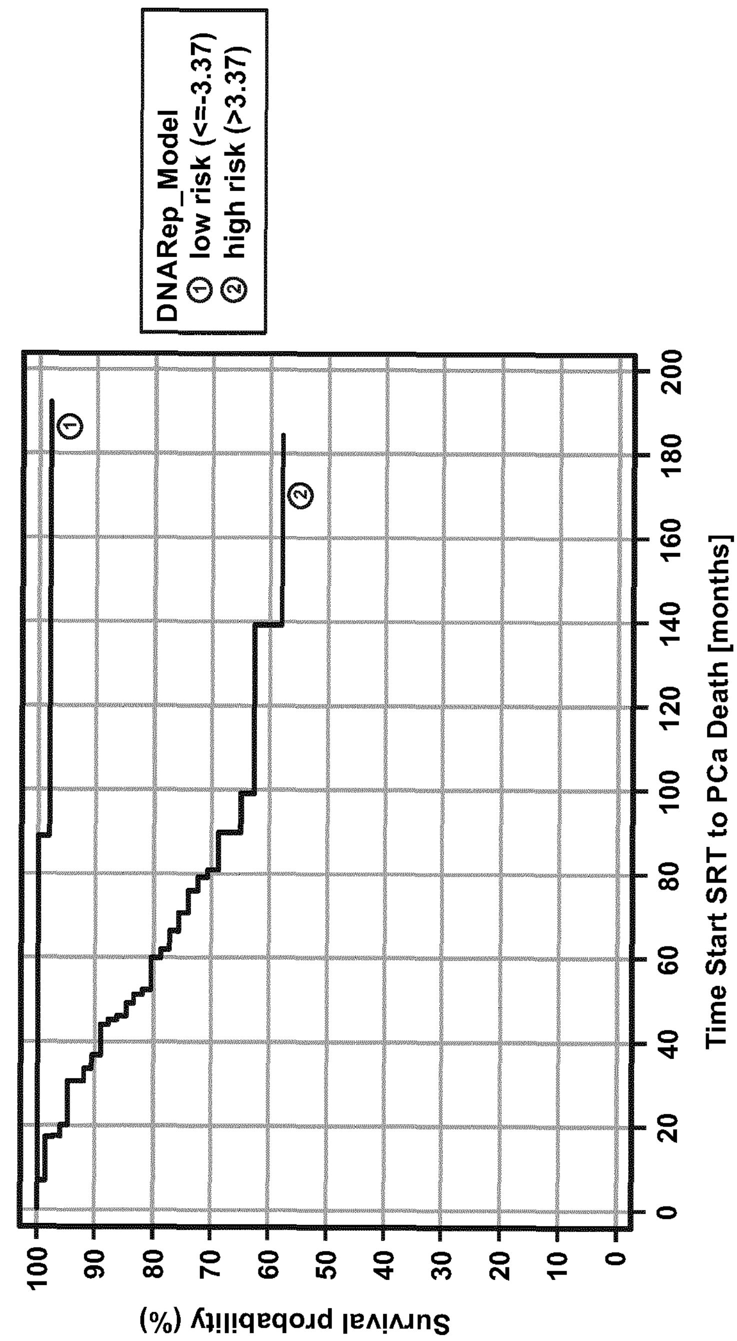
DNARep_Model
① low risk (<=-3.37)
② high risk (>3.37)
Survival probability (%)
100
90
80
70
60
50
40
30
20
10
0
0
20
40
60
80
100
120
140
160
180
200
Time Start SRT to PCa Death [months]
①
②

FIG. 5

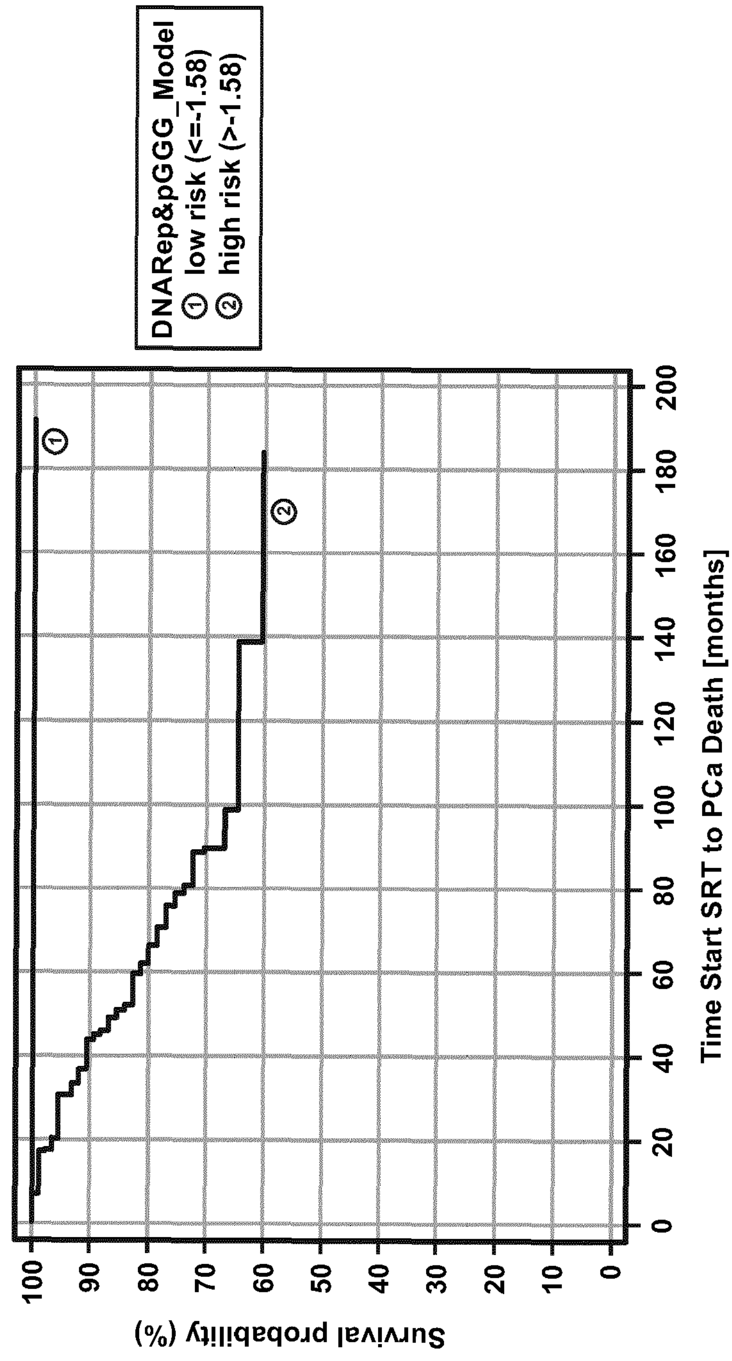
DNARep&pGGG_Model
① low risk (<=-1.58)
② high risk (>-1.58)
①
②
Survival probability (%)
100
90
80
70
60
50
40
30
20
10
0
0
20
40
60
80
100
120
140
160
180
200
Time Start SRT to PCa Death [months]

FIG. 6

Europäisches Patentamt
European Patent Office
Office européen des brevets

# EUROPEAN SEARCH REPORT

Application Number
EP 20 16 4829

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Affymetrix GeneChip Human Genome U133 Array Set HGU133A", NCBI, GEO, Platform GPL96, 11 March 2002 (2002-03-11), pages 1-511, XP055546924, Retrieved from the Internet: URL:https://www.ncbi.nlm.nih.gov/geo/query/acc.cgi?acc=GPL96 [retrieved on 2019-01-24] * the whole document * ----- | 12,13,15 | INV. C12Q1/6886 |
| Y | ROBERT B. DEN ET AL: "Genomic Classifier Identifies Men With Adverse Pathology After Radical Prostatectomy Who Benefit From Adjuvant Radiation Therapy", JOURNAL OF CLINICAL ONCOLOGY, vol. 33, no. 8, 10 March 2015 (2015-03-10), pages 944-951, XP055592410, US ISSN: 0732-183X, DOI: 10.1200/JCO.2014.59.0026 * the whole document * ----- | 1-16 | TECHNICAL FIELDS SEARCHED (IPC) |
| Y | CLEMENS KRATOCHWIL ET AL: "Patients Resistant Against PSMA-Targeting [alpha]-Radiation Therapy Often Harbor Mutations in DNA Damage-Repair-Associated Genes", THE JOURNAL OF NUCLEAR MEDICINE, vol. 61, no. 5, 10 October 2019 (2019-10-10), pages 683-688, XP55733970, US ISSN: 0161-5505, DOI: 10.2967/jnumed.119.234559 * the whole document * ----- -/-- | 1-16 | C12Q |

The present search report has been drawn up for all claims

1

| Place of search | Date of completion of the search | Examiner |
|---|---|---|
| The Hague | 25 September 2020 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 16 4829

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHEN XIAODONG ET AL: "IL-6 signaling contributes to radioresistance of prostate cancer through key DNA repair-associated molecules ATM, ATR, and BRCA 1/2", JOURNAL OF CANCER RESEARCH AND CLINICAL ONCOLOGY, SPRINGER INTERNATIONAL, BERLIN, DE, vol. 145, no. 6, 24 April 2019 (2019-04-24), pages 1471-1484, XP036786948, ISSN: 0171-5216, DOI: 10.1007/S00432-019-02917-Z [retrieved on 2019-04-24] * the whole document * ----- | 1-16 | |
| A | NICHOLAS ERHO ET AL: "Discovery and Validation of a Prostate Cancer Genomic Classifier that Predicts Early Metastasis Following Radical Prostatectomy", PLOS ONE, vol. 8, no. 6, 24 June 2013 (2013-06-24), page e66855, XP055371391, DOI: 10.1371/journal.pone.0066855 ----- | 1-16 | |

TECHNICAL FIELDS SEARCHED (IPC)

The present search report has been drawn up for all claims

1

| Place of search | Date of completion of the search | Examiner |
|---|---|---|
| The Hague | 25 September 2020 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int,* vol. 1, 22-29, 2012
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 2016, vol. 27, 11-20
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **KANG Z. et al.** DNA repair in cancer. *J Oncol,* vol. 2019
- **COOPERBERG M.R. et al.** The CAPRA-S score: A straightforward tool for improved prediction of outcomes after radical prostatectomy. *Cancer,* 2011, vol. 117 (22), 5039-5046